(19) Europäisches Patentamt
European Patent Office
Office européen des brevets

(11) EP 1 414 837 B1

(12) EUROPEAN PATENT SPECIFICATION

(45) Date of publication and mention of the grant of the patent: **19.10.2005 Bulletin 2005/42**

(21) Application number: **02763415.3**

(22) Date of filing: **06.08.2002**

(51) Int Cl.[7]: **C07H 19/16**, A61K 31/70, A61P 35/00

(86) International application number: **PCT/US2002/024696**

(87) International publication number: **WO 2003/014137 (20.02.2003 Gazette 2003/08)**

# (54) ADENOSINE A3 RECEPTOR AGONISTS

AGONISTEN FÜR DEN ADENOSIN A3 REZEPTOR

AGONISTES DU RECEPTEUR ADENOSINE A3

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **08.08.2001 US 311069 P**

(43) Date of publication of application: **06.05.2004 Bulletin 2004/19**

(73) Proprietor: **CV THERAPEUTICS, INC.**
**Palo Alto, CA 94304 (US)**

(72) Inventors:
- **ELZEIN, Elfatih**
  **Fremont, CA 94555 (US)**
- **PALLE, Venkata**
  **Gurgoan 122001 (IN)**
- **VARKHEDKAR, Vaibhav**
  **Mountain View, CA 94040 (US)**
- **ZABLOCKI, Jeff**
  **Mountain View, CA 94040 (US)**

(74) Representative: **Schnappauf, Georg**
**Dr. Volker Vossius**
**Patent- und Rechtsanwaltskanzlei**
**Geibelstrasse 6**
**81679 München (DE)**

(56) References cited:
**WO-A-00/78777** **WO-A-00/78778**
**WO-A-00/78779**

- **KLOTZ K-N ET AL: "2-SUBSTITUTED N-ETHYLCARBOXAMIDOADENOSINE DERIVATIVES AS HIGH-AFFINITY AGONISTS AT HUMAN A3 ADENOSINE RECEPTORS" NAUNYN-SCHMIEDEBERG'S ARCHIVES OF PHARMACOLOGY, SPRINGER, BERLIN, DE, vol. 360, no. 2, 1999, pages 103-108, XP000984051 ISSN: 0028-1298**
- **BARALDI P G ET AL: "NOVEL N6-(SUBSTITUTED-PHENYLCARBAMOYL)ADENOSINE- 5'-URONAMIDES AS POTENT AGONIST FOR A3 ADENOSINE RECEPTORS" JOURNAL OF MEDICINAL CHEMISTRY, AMERICAN CHEMICAL SOCIETY. WASHINGTON, US, vol. 39, no. 3, February 1996 (1996-02), pages 802-806, XP002913657 ISSN: 0022-2623**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 414 837 B1

## Description

### Field of the Invention

This invention relates to novel adenosine $A_3$ receptor agonists that are useful in the treatment of neurological, cardiac, and other cellular proliferative disorders. The invention also relates to methods for the preparation of such compounds, and to pharmaceutical compositions containing them.

### Background

Adenosine is a naturally occurring nucleoside, which exerts its biological effects by interacting with a family of adenosine receptors known as $A_1$, $A_{2a}$, $A_{2b}$, and $A_3$, all of which modulate important physiological processes. For example, stimulation of the $A_1$ adenosine receptors shortens the duration and decreases the amplitude of the action potential of AV nodal cells, and hence prolongs the refractory period of the AV nodal celL Thus, stimulation of $A_1$ receptors provides a method of treating supraventricular tachycardias, including termination of nodal re-entrant tachycardias, and control of ventricular rate during atrial fibrillation and flutter. $A_{2A}$ adenosine receptors modulate coronary vasodilation, $A_{2B}$ receptors have been implicated in mast cell activation, asthma, vasodilation, regulation of cell growth, intestinal function, and modulation of neurosecretion (See Adenosine $A_{2B}$ Receptors as Therapeutic Targets, Drug Dev Res 45:198; Feoktistov et al.., Trends Pharmacol Sci 19:148-153). $A_3$ adenosine receptors modulate cell proliferation processes. In particular, compounds that are $A_3$ receptor agonists have utility in the therapeutic and/or prophylactic treatment of cancer, cardiac disease, infertility, kidney disease, and CNS disorders. Additionally, $A_3$ receptor agonists stimulate bone marrow cell proliferation, and thus induce the secretion of G-CSF in the body. Accordingly, $A_3$ receptor agonists are useful for countering the toxic side effect of drugs, in particular chemotherapeutic drugs, such as leukopenia and neutropenia.

Few ligands for the $A_3$ adenosine receptor have been reported. Some non-selective $N^6$ - substituted adenosine derivatives have been described as agonists for the $A_3$ receptor, including APNEA ($N^6$ -2-(4-aminophenyl)ethyladenosine), which has been used successfully as a radioligand in its iodinated form (Zhou et al. ,Proc. Natl. Acad. Sci. USA, 1992, 89, 7432-7436). Typical xanthine and nonxanthine $A_1$ and $A_2$ receptor antagonists, however, do not appear to bind to this receptor (Zhou et al. ,Proc. Natl. Acad. Sci. USA, 1992, 89, 7432-7436).

Klotz et al., Naunyn-Schmiedeberg's Arch. Pharmacol. 1999, 360, 103-108 describe 2-substituted *N*-ethylcarboxamidoadenosine derivatives as high-affinity agonists at human $A_3$ adenosine receptors.

Baraldi et al., J. Med. Chem. 1996, 39, 802-806 disclose $N^6$-(substituted-phenylcarbamoyl)adenosine-5'-uronamides as potent agonists for $A_3$ adenosine receptors.

WO 00/78777 describes 2-adenosine propargyl phenyl ether compounds that are useful as $A_{2A}$ receptor agonists.

WO 00/78778 discloses 2-adenosine C-pyrazole compounds that are useful as $A_{2A}$ receptor agonists.

WO 00/78779 describes 2-adenosine N-pyrazole compounds that are useful as $A_{2A}$ receptor agonists.

Accordingly, it is desired to provide compounds that are $A_3$ receptor agonists. Preferably, the compounds would be selective for the $A_3$ receptor, thus avoiding side effects caused by interaction with other adenosine receptors.

### SUMMARY OF THE INVENTION

It is an object of this invention to provide $A_3$ receptor agonists. Accordingly, in a first aspect, the invention relates to compounds of Formula I:

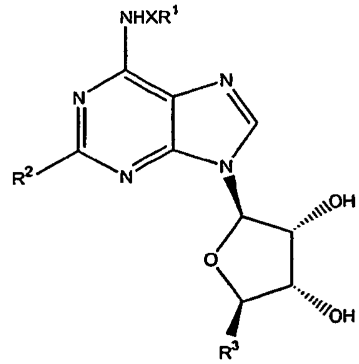

Formula I

wherein:

$R^1$ is optionally substituted lower alkyl, optionally substituted cycloalkyl, optionally substituted aryl, or optionally substituted heteroaryl;
X is a covalent bond or optionally substituted alkylene;
$R^2$ is $R^4$-Z-Y-C≡C- or optionally substituted pyrazolyl:

in which Y is optionally substituted alkylene, Z is oxygen, sulfur or -NH-, and $R^4$ is optionally substituted aryl or optionally substituted heteroaryl; and

$R^3$ is hydroxymethyl or -C(O)-$NR^5R^6$;
in which $R^5$ and $R^6$ are independently hydrogen or lower alkyl;

and the pharmaceutically acceptable salts, esters and prodrugs thereof

A second aspect of this invention relates to pharmaceutical formulations, comprising a therapeutically effective amount of a compound of Formula I and at least one pharmaceutically acceptable excipient.

A third aspect of this invention relates to a method of using the compounds of Formula I in the treatment of a disease or condition in a mammal that can be usefully treated with an $A_3$ receptor agonist, comprising administering to a mammal in need thereof a therapeutically effective dose of a compound of Formula I. Such diseases include, but are not limited to, cancer, renal and cardiac ischemia, neurodegenerative disorders, infertility, neutropenia, kidney disease, and CNS disorders.

A fourth aspect of this invention relates to methods of preparing the compounds of Formula I.

Of the compounds of Formula I, one preferred class includes those in which $R^2$ is optionally substituted pyrazol-1-yl, especially where $R^1$ is optionally substituted alkyl or optionally substituted aryl, $R^3$ is hydroxymethyl, and X is a covalent bond. Of these compounds, one preferred group includes those compounds in which $R^2$ is pyrazol-1-yl substituted by optionally substituted lower alkyl, ester, aminocarbonyl, optionally substituted aryl, or optionally substituted heteroaryl.

A preferred subgroup includes those compounds in which $R^2$ is pyrazol-1-yl substituted by optionally substituted phenyl or optionally substituted alkyl, and $R^1$ is optionally substituted alkyl. More preferred are those compounds in which $R^1$ is lower alkyl of 1-3 carbon atoms and $R^2$ is pyrazol-1-yl substituted by phenyl or benzyl having methoxy or chloro substitutions.

A second preferred subgroup includes those compounds in which $R^2$ is pyrazol-1-yl substituted by optionally substituted heteroaryl and $R^1$ is optionally substituted alkyl. More preferred are those compounds in which $R^1$ is lower alkyl of 1-3 carbon atoms and $R^2$ is pyrazol-1-yl substituted by pyridine.

A third preferred subgroup includes those compounds in which $R^2$ is pyrazol-1-yl substituted by optionally substituted phenyl, $R^1$ is optionally substituted phenyl, and X is alkylene. More preferred are those compounds in which $R^1$ is 3-iodophenyl, especially where X is methylene.

A second preferred class includes compounds in which $R^2$ is pyrazol-4-yl optionally substituted by optionally substituted phenyl or optionally substituted alkyl, especially where $R^1$ is optionally substituted alkyl and X is a covalent bond. More preferred are those compounds in which $R^1$ is lower alkyl.

A third preferred class includes those compounds in which $R^2$ is $R^4$-Z-Y-C≡C-, especially where $R^4$ is op-

tionally substituted phenyl and Y is alkylene of 1, 2, or 3 carbon atoms. Of these compounds, a preferred group includes those compounds in which $R^4$ is phenyl optionally substituted by methoxy or chloro, and Y is methylene, $R^1$ is optionally substituted lower alkyl, $R^3$ is hydroxy, X is a covalent bond, and Z is oxygen.

## Definitions and General Parameters

As used in the present specification, the following words and phrases are generally intended to have the meanings as set forth below, except to the extent that the context in which they are used indicates otherwise.

The term "alkyl" refers to a monoradical branched or unbranched saturated hydrocarbon chain having from 1 to 20 carbon atoms. This term is exemplified by groups such as methyl, ethyl, n-propyl, iso-propyl, n-butyl, iso-butyl, t-butyl, n-hexyl,n-decyl, tetradecyl, and the like.

The term "substituted alkyl" refers to:

1) an alkyl group as defined above, having 1, 2, 3, 4 or 5 substituents, preferably 1 to3 substituents, selected from the group consisting of alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, $-SO_2$-alkyl, $SO_2$-aryl and $-SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2; or

2) an alkyl group as defined above that is interrupted by 1-10 atoms independently chosen from oxygen, sulfur and $NR_a$-, where $R_a$ is chosen from hydrogen, alkyl, cycloalkyl, alkenyl, cycloalkenyl, alkynyl, aryl, heteroaryl and heterocyclyl. All substituents may be optionally further substituted by alkyl, alkoxy, halogen, $CF_3$, amino, substituted amino, cyano, or $-S(O)_nR$, in which R is alkyl, aryl, or heteroaryl and n is 0, 1 or 2; or

3) an alkyl group as defined above that has both 1, 2, 3, 4 or 5 substituents as defined above and is also interrupted by 1-10 atoms as defined above.

The term "lower alkyl" refers to a monoradical branched or unbranched saturated hydrocarbon chain having 1, 2, 3, 4, 5, or 6carbon atoms. This term is exemplified by groups such as methyl, ethyl, n-propyl, iso-propyl, n-butyl, iso-butyl, t-butyl, n-hexyl, and the like.

The term "substituted lower alkyl" refers to lower alkyl as defined above having 1 to 5 substituents, preferably 1, 2, or 3 substituents, as defined for substituted alkyl, or a lower alkyl group as defined above that is interrupted by 1, 2, 3, 4, or 5 atoms as defined for substituted alkyl, or a lower alkyl group as defined above that has both 1, 2, 3, 4 or 5 substituents as defined above and is also interrupted by 1, 2, 3, 4, or 5 atoms as defined above.

The term "alkylene" refers to a diradical of a branched or unbranched saturated hydrocarbon chain, preferably having from 1 to 20 carbon atoms, preferably 1-10 carbon atoms, more preferably 1, 2, 3, 4, 5 or 6 carbon atoms. This term is exemplified by groups such as methylene ($-CH_2-$), ethylene ($-CH_2CH_2-$), the propylene isomers (e.g., $-CH_2CH_2CH_2-$ and-$CH(CH_3)CH_2-$) and the like.

The term "lower alkylene" refers to a diradical of a branched or unbranched saturated hydrocarbon chain, preferably having from 1, 2, 3, 4, 5, or 6 carbon atoms.

The term"substituted alkylene" refers to:

(1) an alkylene group as defined above having 1, 2, 3, 4, or 5 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, $-SO_2$-alkyl, $SO_2$-aryl and $-SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2; or

(2) an alkylene group as defined above that is interrupted by 1-20atoms independently chosen from oxygen, sulfur and $NR_a$-, where $R_a$ is chosen from hydrogen, optionally substituted alkyl, cycloalkyl, cycloalkenyl, aryl, heteroaryl and heterocycyl, or groups selected from carbonyl, carboxyester, carboxyamide and sulfonyl; or

(3) an alkylene group as defined above that has both 1, 2, 3, 4 or 5 substituents as defined above and is also interrupted by 1-20 atoms as defined above. Examples of substituted alkylenes are chloromethylene (-CH(Cl)-), aminoethylene ($-CH(NH_2)CH_2-$), methylaminoethylene ($-CH(NHMe)CH_2-$), 2-carboxypropylene isomers($-CH_2CH$

$(CO_2H)CH_2$-), ethoxyethyl ($-CH_2CH_2O-CH_2CH_2-$), ethylmethylaminoethyl ($-CH_2CH_2N(CH_3)CH_2CH_2-$),1-ethoxy-2-(2-ethoxy-ethoxy)ethane ($-CH_2CH_2O-CH_2CH_2-OCH_2CH_2-OCH_2CH_2-$), and the like.

The term "aralkyl" refers to an aryl group covalently linked to an alkylene group, where aryl and alkylene are defined herein. "Optionally substituted aralkyl" refers to an optionally substituted aryl group covalently linked to an optionally substituted alkylene group. Such aralkyl groups are exemplified by benzyl, phenylethyl, 3-(4-methoxyphenyl) propyl, and the like.

The term "alkoxy" refers to the group R-O-, where R is optionally substituted alkyl or optionally substituted cycloalkyl, or R is a group -Y-Z, in which Y is optionally substituted alkylene and Z is optionally substituted alkenyl, optionally substituted alkynyl; or optionally substituted cycloalkenyl, where alkyl, alkenyl, alkynyl, cycloalkyl and cycloalkenyl are as defined herein. Preferred alkoxy groups are alkyl-O- and include, by way of example, methoxy, ethoxy, n-propoxy, iso-propoxy, n-butoxy, tert-butoxy, sec-butoxy, n-pentoxy, n-hexoxy, 1,2-dimethylbutoxy, and the like.

The term "alkylthio" refers to the group R-S-, where R is as defined for alkoxy.

The term "alkenyl" refers to a monoradical of a branched or unbranched unsaturated hydrocarbon group preferably having from 2 to 20 carbon atoms, more preferably 2 to 10 carbon atoms and even more preferably 2 to 6 carbon atoms and having 1-6, preferably 1, double bond (vinyl). Preferred alkenyl groups include ethenyl or vinyl ($-CH=CH_2$), 1-propylene or allyl ($-CH_2CH=CH_2$), isopropylene ($-C(CH_3)=CH_2$), bicyclo[2.2.1]heptene, and the like. In the event that alkenyl is attached to nitrogen, the double bond cannot be alpha to the nitrogen.

The term "lower alkenyl" refers to alkenyl as defined above having from 2 to 6 carbon atoms.

The term "substituted alkenyl" refers to an alkenyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, $-SO_2$-alkyl, $SO_2$-aryl and $-SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2.

The term "alkynyl" refers to a monoradical of an unsaturated hydrocarbon, preferably having from 2 to 20 carbon atoms, more preferably 2 to 10 carbon atoms and even more preferably 2 to 6 carbon atoms and having at least 1 and preferably from 1-6 sites of acetylene (triple bond) unsaturation. Preferred alkynyl groups include ethynyl, ($-C\equiv CH$), propargyl (or propynyl, $-C\equiv CCH_3$), and the like. In the event that alkynyl is attached to nitrogen, the triple bond cannot be alpha to the nitrogen.

The term "substituted alkynyl" refers to an alkynyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aininosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, $-SO_2$-alkyl, $SO_2$-aryl and $-SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2.

The term "aminocarbonyl" refers to the group -C(O)NRR where each R is independently hydrogen, alkyl, cycloaklyl, aryl, heteroaryl, heterocyclyl or where both R groups are joined to form a heterocyclic group (e.g., morpholino). Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2.

The term "ester" or "carboxyester" refers to the group -C(O)OR, where R is alkyl, cycloalkyl, aryl, heteroaryl, or heterocyclyl, which may be optionally further substituted by alkyl, alkoxy, halogen, $CF_3$, amino, substituted amino, cyano, or $-S(O)_nR_a$, in which $R_a$ is alkyl, aryl, or heteroaryl and n is 0, 1 or 2.

The term "acylamino" refers to the group -NRC(O)R where each R is independently hydrogen, alkyl, aryl, heteroaryl, or heterocyclyl. All substituents may be optionally further substituted by alkyl, alkoxy, halogen, $CF_3$, amino, substituted amino, cyano, or $-S(O)_nR$, in which R is alkyl, aryl, or heteroaryl and n is 0, 1 or 2.

The term "acyloxy" refers to the groups -O(O)C-alkyl, -O(O)C-cycloalkyl, -O(O)C-aryl, -O(O)C-heteroaryl, and -O(O)C-heterocyclyl. Unless otherwise constrained by the definition, all substituents may optionally be further substituted by 1,2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2.

The term "aryl" refers to an aromatic carbocyclic group of 6 to 20 carbon atoms having a single ring (e.g.,

phenyl) or multiple rings (e.g., biphenyl), or multiple condensed (fused) rings (e.g., naphthyl or anthryl). Preferred aryls include phenyl, naphthyl and the like.

Unless otherwise constrained by the definition for the aryl substituent, such aryl groups can optionally be substituted with 1, 2, 3, 4 or 5 substituents, preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -$SO_2$-alkyl, $SO_2$-aryl and -$SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and -$S(O)_nR$, where R is alkyl, aryl, or heteroaryl and n is 0, 1 or 2.

The term "aryloxy" refers to the group aryl-O- wherein the aryl group is as defined above, and includes optionally substituted aryl groups as also defined above. The term "arylthio" refers to the group R-S-, where R is as defined for aryl.

The term "amino" refers to the group -$NH_2$.

The term "substituted amino" refers to the group -NRR where each R is independently selected from the group consisting of hydrogen, alkyl, cycloalkyl, aryl, heteroaryl and heterocyclyl provided that both R groups are not hydrogen, or a group -Y-Z, in which Y is optionally substituted alkylene and Z is alkenyl, cycloalkenyl, or alkynyl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and -$S(O)_nR$, where R is alkyl, aryl, or heteroaryl and n is 0,1 or 2.

The term "carboxyalkyl" refers to the groups -C(O)O-alkyl, -C(O)O-cycloalkyl, where alkyl and cycloalkyl, are as defined herein, and may be optionally further substituted by alkyl, alkenyl, alkynyl, alkoxy, halogen, $CF_3$, amino, substituted amino, cyano, or -$S(O)_nR$, in which R is alkyl, aryl, or heteroaryl and n is 0, 1 or 2.

The term "cycloalkyl" refers to cyclic alkyl groups of from 3 to 20 carbon atoms having a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl, and bicyclo[2.2.1]heptane, or cyclic alkyl groups to which is fused an aryl group, for example indan, and the like.

The term "substituted cycloalkyl" refers to cycloalkyl groups having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -$SO_2$-alkyl, $SO_2$-aryl and -$SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and -$S(O)_nR$, where R is alkyl, aryl, or heteroaryl and n is 0, 1 or 2.

The term "halogen" or "halo" refers to fluoro, bromo, chloro, and iodo.

The term "acyl" denotes a group -C(O)R, in which R is hydrogen, optionally substituted alkyl, optionally substituted cycloalkyl, optionally substituted heterocyclyl, optionally substituted aryl, and optionally substituted heteroaryl.

The term "heteroaryl" refers to an aromatic group (i.e., unsaturated) comprising 1 to 15 carbon atoms and 1 to 4 heteroatoms selected from oxygen, nitrogen and sulfur within at least one ring.

Unless otherwise constrained by the definition for the heteroaryl substituent, such heteroaryl groups can be optionally substituted with 1 to 5 substituents, preferably 1, 2, or 3 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl (an alkyl ester), arylthio, heteroaryl, heteroarylthio, heterocyclylthio, thiol, alkylthio, aryl, aryloxy, aralkyl, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -$SO_2$-alkyl, $SO_2$-aryl and -$SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and -$S(O)_nR$, where R is alkyl, aryl, or heteroaryl and n is 0, 1 or 2. Such heteroaryl groups can have a single ring (e.g., pyridyl or furyl) or multiple condensed rings (e.g., indolizinyl, benzothiazole, or benzothienyl). Examples of nitrogen heterocycles and heteroaryls include, but are not limited to, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, and the like as well as N-alkoxy-nitrogen containing heteroaryl compounds.

One choice for the definition of $R^4$ in Formula I is a heteroaryl, namely an optionally substituted pyrazole. This

definition is intended to include pyrazoles attached:

a) through the N1 position of the pyrazole, that is a pyrazol-1-yl moiety of the formula:

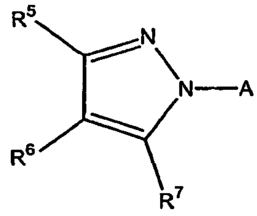

in which A represents the point of attachment to the 2-position of the compound of Formula I, and $R^5$, $R^6$, and $R^7$ are independently hydrogen or those optional substitutions shown for heteroaryl above; and
b) through any carbon atom of the pyrazole, that is a C-pyrazolyl of the formula:

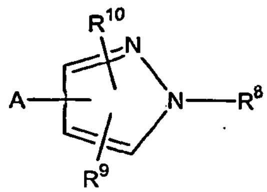

in which A represents the point of attachment to the 2-position of the compound of Formula I, and $R^8$, $R^9$ and, $R^{10}$ are independently hydrogen or those optional substitutions shown for heteroaryl above.

Preferred are optionally substituted pyrazol-1-yl and optionally substituted pyrazol-4-yl. Preferred substitutions are hydrogen, optionally substituted aryl, optionally substituted aralkyl, optionally substituted heteroaryl, and optionally substituted heteroaralkyl.

The term "heteroaryloxy" refers to the group heteroaryl-O-.

The term "heterocyclyl" refers to a monoradical saturated or partially unsaturated group having a single ring or multiple condensed rings, having from 1 to 40 carbon atoms and from 1 to 10 hetero atoms, preferably 1 to 4 heteroatoms, selected from nitrogen, sulfur, phosphorus, and/or oxygen within the ring.

Unless otherwise constrained by the definition for the heterocyclic substituent, such heterocyclic groups can be optionally substituted with 1 to 5, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, $-SO_2$-alkyl, $SO_2$-aryl and $-SO_2$-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, $CF_3$, amino, substituted amino, cyano, and $-S(O)_nR$, where R is alkyl, aryl, or heteroaryl and n is 0, 1 or 2. Heterocyclic groups can have a single ring or multiple condensed rings. Preferred heterocyclics include tetrahydrofuranyl, morpholino, piperidinyl, and the like.

The term "thiol" refers to the group -SH.

The term "substituted alkylthio" refers to the group -S-substituted alkyl.

The term "heteroarylthiol" refers to the group -S-heteroaryl wherein the heteroaryl group is as defined above including optionally substituted heteroaryl groups as also defined above.

The term "sulfoxide" refers to a group -S(O)R, in which R is alkyl, aryl, or heteroaryl. "Substituted sulfoxide" refers to a group -S(O)R, in which R is substituted alkyl, substituted aryl, or substituted heteroaryl, as defined herein.

The term "sulfone" refers to a group $-S(O)_2R$, in which R is alkyl, aryl, or heteroaryl. "Substituted sulfone" refers to a group $-S(O)_2R$, in which R is substituted alkyl, substituted aryl, or substituted heteroaryl, as defined herein.

The term "keto" refers to a group -C(O)-. The term "thiocarbonyl" refers to a group-C(S)-. The term "carboxy" refers to a group -C(O)-OH.

"Optional" or "optionally" means that the subsequently described event or circumstance may or may not occur, and that the description includes instances where said event or circumstance occurs and instances in which it does not.

The term "compound of Formula I" is intended to encompass the compounds of the invention as disclosed,

and the pharmaceutically acceptable salts, pharmaceutically acceptable esters, and prodrugs of such compounds.

The term "therapeutically effective amount" refers to that amount of a compound of Formula I that is sufficient to effect treatment, as defined below, when administered to a mammal in need of such treatment. The therapeutically effective amount will vary depending upon the subject and disease condition being treated, the weight and age of the subject, the severity of the disease condition, the manner of administration and the like, which can readily be determined by one of ordinary skill in the art.

The term "treatment" or "treating" means any treatment of a disease in a mammal, including:

(i) preventing the disease, that is, causing the clinical symptoms of the disease not to devclop;
(ii) inhibiting the disease, that is, arresting the development of clinical symptoms; and/or
(iii) relieving the disease, that is, causing the regression of clinical symptoms.

In many cases, the compounds of this invention are capable of forming acid and/or base salts by virtue of the presence of amino and/or carboxyl groups or groups similar thereto. The term "pharmaceutically acceptable salt" refers to salts that retain the biological effectiveness and properties of the compounds of Formula I, and which are not biologically or otherwise undesirable. Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group.

Specific examples of suitable amines include, by way of example only, isopropylamine, trimethyl amine, diethyl amine, tri(iso-propyl) amine, tri(n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, and the like.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

## Nomenclature

The naming and numbering of the compounds of the invention is illustrated with a representative compound of Formula I in which $R^1$ is methyl, $R^2$ is 4-(4-methoxyphenyl)pyrazol-1-yl, $R^3$ is hydroxymethyl, and X is a covalent bond:

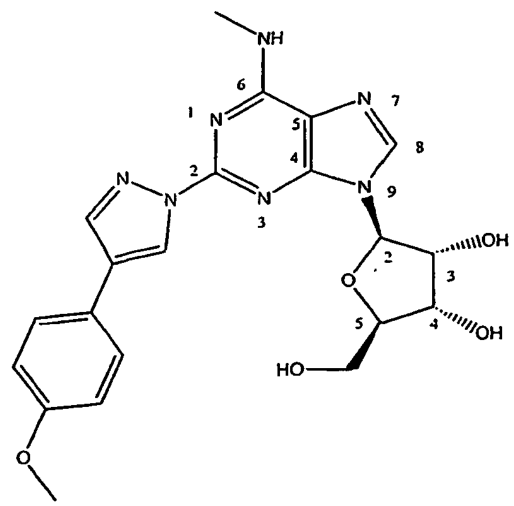

which is named:
(4S,2R,3R,5R)-5-(hydroxymethyl)-2-{2-[4-(4-methoxyphenyl)pyrazolyl]-6-(methylamino)purin-9-yl}oxolane-3,4-diol

Synthesis of the Compounds of Formula I

An example of a method for preparing the compounds of Formula I where $R^2$ is optionally substituted pyrazol-1-yl is shown in Reaction Scheme I.

## REACTION SCHEME I

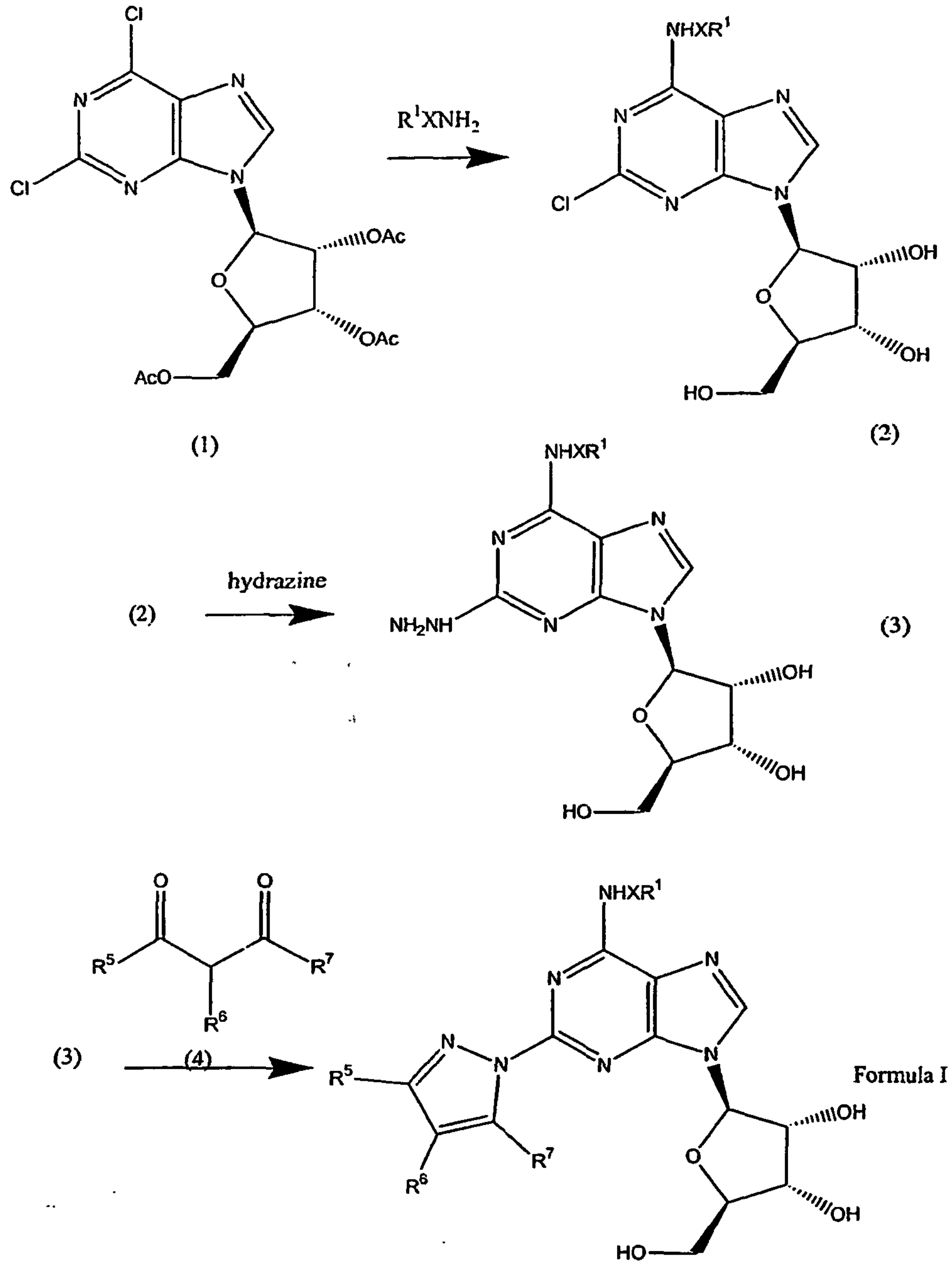


(1) (2) (2) (3) (3) (4) Formula I

where Ac is acetyl, and X is a covalent bond or optionally substituted alkylene.

Step 1 - Preparation of Formula (2)

The compound of formula (2) is prepared by displacement of the 6-chloro of a compound of formula (1), which is prepared as described in J.F. Gorster and R.K. Robins, J. Org. Chem. 1966, Vol. 31,3258-62. The compound of formula (1) is reacted with a compound of formula $R^1XNH_2$, where X is a covalent bond or optionally substituted alkylene, in the presence of a base. The reaction is carried out in an inert protic solvent, for example methanol, ethanol, n-butanol, and the like, at a temperature of between room temperature and about reflux, for about 12-48 hours. When the reaction is substantially complete, the product of formula (2) is isolated by conventional means, for example by removal of the solvent under reduced pressure, followed by chromatography of the residue on silica gel.

Step 2 - Preparation of Formula (3)

The compound of formula (2) is converted to a compound of formula (3) by reaction with hydrazine hydrate. The reaction is carried out with no solvent, or optionally in a protic solvent, for example ethanol, at a temperature of between room temperature and about reflux, for about 12-48 hours.. When the reaction is substantially complete, the product of formula (3) is isolated by conventional means, for example by removal of solvent under reduced pressure and triturating the product with ether. Altematively, the compound of Formula (3) is used in the next step without purification.

Step 3 - Preparation of Formula I

The compound of formula (3) is converted to a compound of Formula I by reaction with an optionally substituted 1,3-propanedione derivative of formula (4). The reaction is carried out by suspending the compound of formula (3) in a protic solvent, preferably ethanol, adding the compound of formula (4), and refluxing the mixture for about 2-16 hours. When the reaction is substantially complete, the product of Formula I is isolated by conventional means, for example filtering off the product.

For example, starting with a compound of formula (4) in which $R^5$ and $R^7$ are hydrogen and $R^6$ is 4-methoxyphenyl provides a compound of Formula I in which $R^2$ is 4-methoxyphenylpyrazol-1-yl. Starting with a compound of formula (4) in which $R^5$ and $R^7$ are hydrogen and $R^6$ is $-CO_2Et$ provides a compound of Formula I in which $R^2$ is 4-ethoxycarbonylpyrazol-1-yl. This ester group can then be hydrolyzed under basic conditions to give the free acid, which in turn can be converted to acid derivatives such as optionally substituted amide by means well known to those skilled in the art, or by the method shown in Reaction Scheme IA.

REACTION SCHEME IA

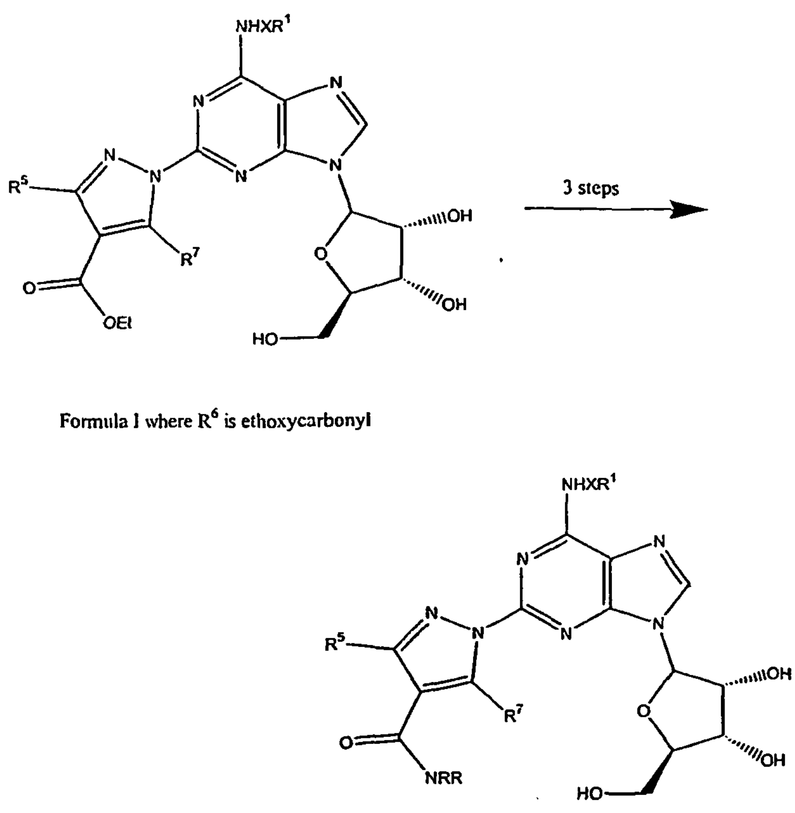


Formula I where $R^6$ is ethoxycarbonyl

Formula I where $R^6$ is amido

Step 1 - Protection of the compound of Formula I where $R^6$ is Ethoxycarbonyl

The compound of Formula I in which $R^6$ is ethoxycarbonyl is dissolved in a polar solvent, preferably DMF, and imidazole and tertiary butyldimethylsilyl chloride added. The reaction is carried out at a temperature of 50-100°C, for about 12-48 hours. When the reaction is substantially complete, the product is isolated by conventional means, for example by removal of the solvent under reduced pressure, followed by flash chromatography of the residue on silica gel.

Step 2 - Hydrolysis of the Ethyl Ester to the Carboxylic Acid

The product from Step 1 is suspended in a mixture of water, an alcohol, and a strong base, preferably potassium hydroxide in methanol. The reaction is carried out at a temperature of 0-40°C, preferably about 25°C, for about 2-5 days, preferably about 3 days.. When the reaction is substantially complete, the solvent is removed under reduced pressure, the residue acidified to a pH of about 5, and the product is isolated by conventional means, for example by filtration.

Step 3 - Preparation of an Amide

The product from Step 2 is dissolved in an inert solvent, preferably dichloromethane, to which is added HBTU, HOBt, N-methylmorpholine,a catalytic amount of DMAP, and an optionally substituted amine of formula HNRR, as defined above. The reaction is carried out at a temperature of 0-40°C, preferably about 25°C, for about 8-48 hours, preferably about 24 hours. When the reaction is substantially complete, the product is isolated by conventional means.

Step 4 - Deprotection

The product from Step 2 is treated with a solution of ammonium fluoride in methanol. The reaction is carried out at a temperature of about reflux, for about 8-48 hours, preferably about 24 hours.. When the reaction is substantially complete, the solvent is removed under reduced pressure, the residue acidified to a pH of about 5, and the product is isolated by conventional means, for example by preparative TLC.

A example of a method for preparing the compounds of Formula I where $R^2$ is optionally substituted pyrazol-4-yl is shown in Reaction Scheme II. This method and other general methods of preparation of pyrazol-4-yl derivatives are shown in U.S. Patent No. 6,214,807.

## REACTION SCHEME II

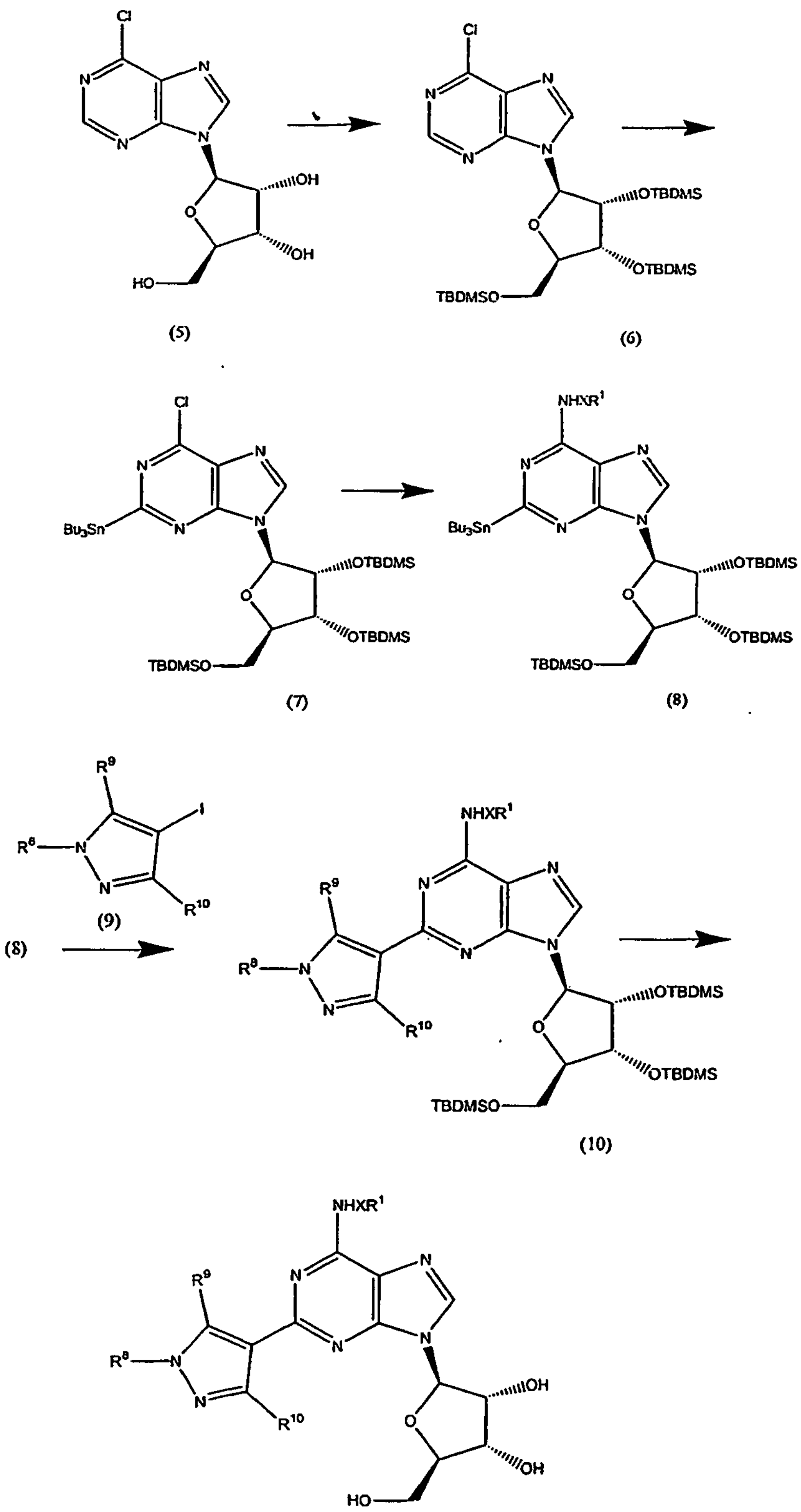

(5) (6)

(7) (8)

(9)

(8)

(10)

Formula I

The starting material of formula (5) is prepared by means well know in the art. The intermediate of formula (9) is prepared as shown below.

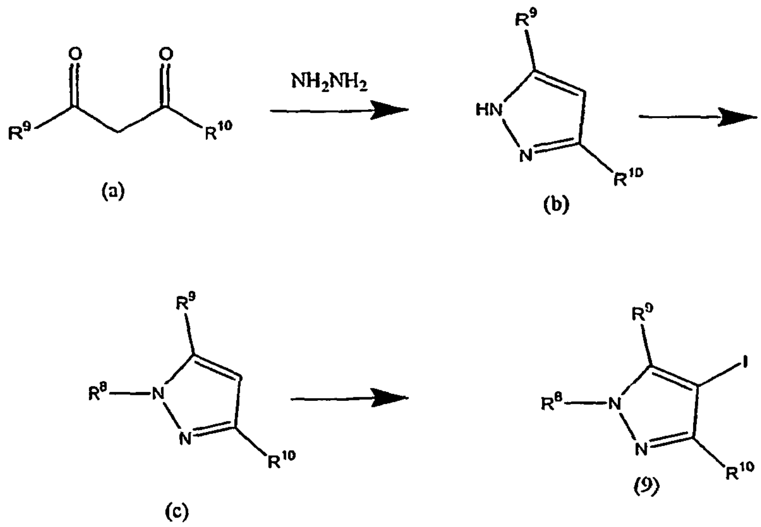


(a) (b) (c) (9)

Condensation of a 1,3-dione of formula (a) with hydrazine in an appropriate solvent provides a pyrazole of formula (b), which is N-alkylated with an appropriate halide of formula $R^8$Hal to give a compound of formula (c). Formation of an anion at the 4-position with a strong base followed by quenching with iodine provides the 4-iodo derivative of formula (9) (F. Effenberger et. al. J. Org. Chem. (1984), 49, 4687).

The iodopyrazole of formula (9) is converted to the corresponding compound of formula (10) by palladium mediated coupling with the compound of formula (8) in the presence or absence of copper salts (K. Kato et. al. J. Org. Chem. 1997, 62, 6833-6841; Palladium Reagents and Catalysts-Innovations in Organic Synthesis, Tsuji, John Wiley and Sons, 1995). The synthesis of the tributyltin derivative of formula (7) has been previously described (K. Kato et. al. J. Org. Chem. 1997, 62, 6833-6841), as shown above in Reaction Scheme II.

An example of a method for preparing the compounds of Formula I where $R^2$ is an ethynyl derivative is shown in Reaction Scheme III.

REACTION SCHEME III

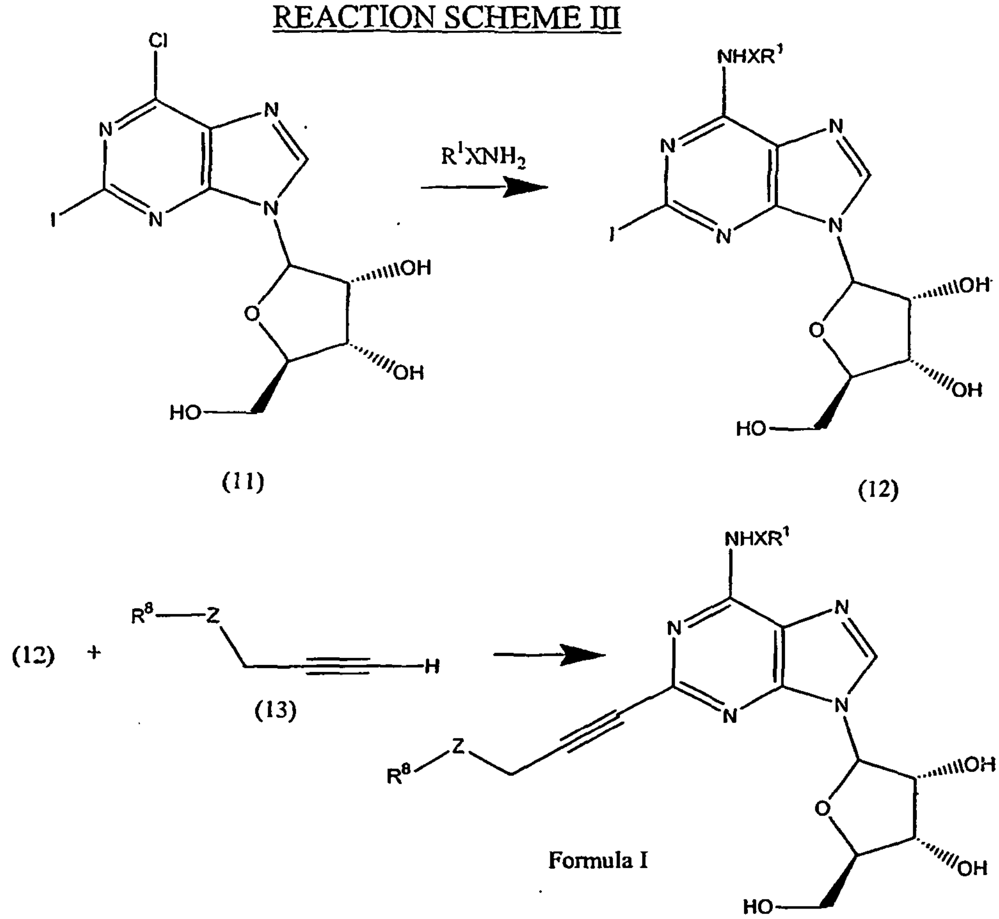


(11) (12)

(12) + (13)

Formula I

where $R^8$ represents optionally substituted aryl or aralkyl and Z is oxygen, sulfur, or -NH-.

Step 1 - Preparation of Formula (12)

The starting compound of formula (11) (2-iodoadenosine) is prepared in four steps from guanosine following literature procedures (M. J. Robins et.al. Can. J. Chem. (1981), 59, 2601-2607; J. F. Cerster et.al. Org. Synthesis, 242-243; V. Nair at. al., J. Org. Chem., (1988), 53, 3051-3057).

The compound of formula (12) is prepared by displacement of the 6-chloro substituent of a compound of formula (11) by reaction with a compound of formula $R^1XNH_2$, where X is a covalent bond or optionally substituted alkylene, in the presence of a base, in the same manner as shown above for the preparation of a compound of formula (2).

Step 2 - Preparation of Formula I

The compound of Formula I where $R^2$ is an ethynyl derivative is prepared from a compound of formula (12) by reaction with an appropriately substituted ethynyl derivative of formula (13). The reaction is carried out in a polar solvent, preferably DMF, in the presence of copper iodide and dichlorobis(triphenylphosphine)palladium(II) catalyst, at a temperature of about 50-100°C, preferably in a sealed tube, for about 2-16 hours. When the reaction is substantially complete, the product of Formula I is isolated by conventional means, for example by thin layer chromatography.

Preferred Processes and Last Steps

The compounds of the present invention can be prepared according to the following last steps:

1. Contacting a compound of the formula:

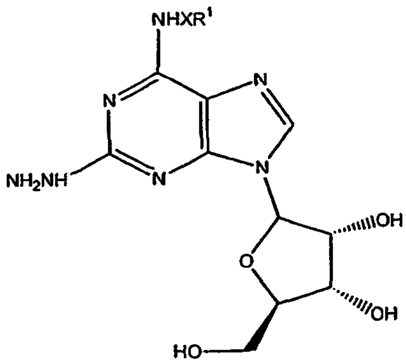

(3)

with a compound of formula:

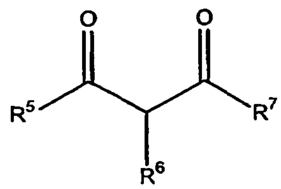

(4)

2. Contacting a compound of the formula:

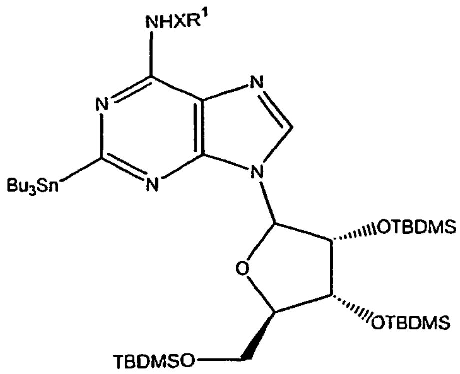

(8)

with a compound of the formula:

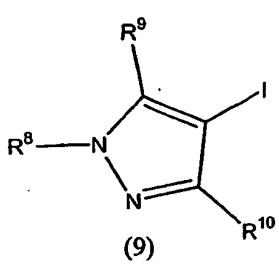

(9)

and contacting the product with a mild acid, for example ammonium fluoride, to remove the protecting groups.

3. Contacting a compound of the formula:

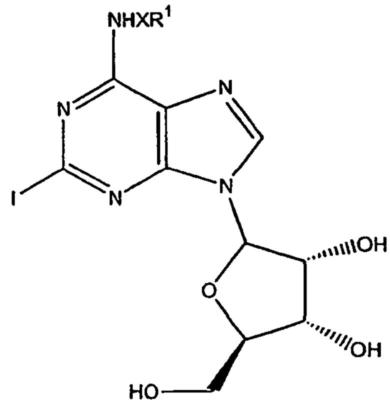

(12)

with a compound of the formula:

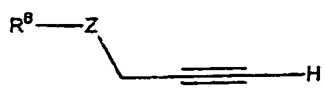

(13)

in the presence of a copper salt and a catalyst.

Utility, Testing and Administration

General Utility

The compounds of Formula I are effective in the treatment of conditions known to respond to administration of $A_3$ adenosine receptor agonists. Such conditions include, but are not limited to, modulation of cell proliferation processes. In particular, compounds that are $A_3$ receptor agonists have utility in the therapeutic and/or prophylactic treatment of cancer, cardiac disease (including use as an ischemia-reperfusion agent), infertility, kidney disease, and CNS disorders. Additionally, they are useful for countering the toxic side effect of drugs, in particular chemotherapeutic drugs, such as leukopenia and neutropenia.

Testing

Activity testing is conducted as described in those patents and patent applications referenced above, and in the Examples below, and by methods apparent to one skilled in the art.

Pharmaceutical Compositions

The compounds of Formula I are usually administered in the form of pharmaceutical compositions. This invention therefore provides pharmaceutical compositions that contain, as the active ingredient, one or more of the compounds of Formula I, or a pharmaceutically acceptable salt or ester thereof, and one or more pharmaceutically acceptable excipients, carriers, including inert solid diluents and fillers, diluents, including sterile aqueous solution and various organic solvents, permeation enhancers, solubilizers and adjuvants. The compounds of Formula I may be administered alone or in combination with other therapeutic agents. Such compositions are prepared in a manner well known in the pharmaceutical art (see, e.g., Remington's Pharmaceutical Sciences, Mace Publishing Co., Philadelphia, PA 17th Ed. (1985) and "Modern Pharmaceutics", Marcel Dekker, Inc. 3rd Ed. (G.S. Banker & C.T. Rhodes, Eds.).

Administration

The compounds of Formula I may be administered in either single or multiple doses by any of the accepted modes of administration of agents having similar utilities, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an artery-inserted cylindrical polymer.

One mode for administration is parental, particularly by injection. The forms in which the novel compositions of the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles. Aqueous solutions in saline are also conventionally used for injection, but less preferred in the context of the present invention. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimemsal, and the like.

Sterile injectable solutions are prepared by incorporating the compound of Formula I in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Oral administration is another route for administration of the compounds of Formula I. Administration may be via capsule or enteric coated tablets, or the like. In making the pharmaceutical compositions that include at least one compound of Formula I, the active ingredient is usually diluted by an excipient and/or enclosed within such a carrier that can be in the form of a capsule, sachet, paper or other container. When the excipient serves as a diluent, it can be a solid, semi-solid, or liquid material (as above), which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents.

The compositions of the invention can be formulated so as to provide quick, sustained or delayed release of the active ingredient after administration to the patient by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Patent Nos. 3,845,770; 4,326,525; 4,902514; and 5,616,345. Another formulation for use in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. *See, e.g.,* U.S. Patent Nos. 5,023,252, 4,992,445 and 5,001,139. Such patches may be constructed for continuous, pulsatile, or on demand delivery of pharmaceutical agents.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient (e.g., a tablet, capsule, ampoule). The compounds of Formula I are effective over a wide dosage range and are generally administered in a pharmaceutically effective amount. Preferably, for oral administration, each dosage unit contains from 10 mg to 2 g of a compound of Formula I, more preferably from 10 to 700 mg, and for parenteral administration, preferably from 10 to 700 mg of a compound of Formula I, more preferably about 50-200 mg. It will be understood, however, that the amount of the compound of Formula I actually administered will be determined by a physician, in the light of the relevant circumstances, including the condition to be treated, the chosen route of administration, the actual compound administered and its relative activity, the age, weight, and response of

the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills of the present invention may be coated or otherwise compounded to provide a dosage form affording the advantage of prolonged action, or to protect from the acid conditions of the stomach. For example, the tablet or pill can comprise an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra*. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner.

The following examples are included to demonstrate preferred embodiments of the invention. It should be appreciated by those of skill in the art that the techniques disclosed in the examples which follow represent techniques discovered by the inventor to function well in the Practice of the invention, and thus can be considered to constitute preferred modes for its practice.

EXAMPLE 1

Preparation of a Compound of Formula (2)

A. Preparation of a Compound of Formula (2) where $R^1$ is Methyl and X is a Covalent Bond

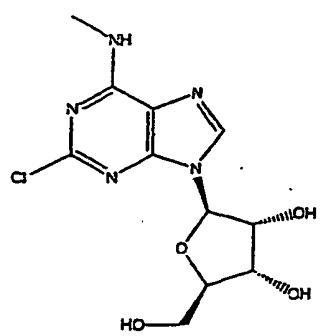

(2)

3,4-diacetyloxy-2-(2,6-dichloropurin-9-yl)-5-(2-oxopropoxy)tetrahydrofuran, the compound of formula (1) (1 mmol), was suspended in a mixture of 1:4 methylamine/MeOH, and the mixture stirred at room temperature for 24 hours. The solvent was removed under reduced pressure and the residue triturated in ether, to afford (4S,2R,3R,5R)-2-[2-chloro-6-(methylamino)purin-9-yl]-5-(hydroxymethyl)oxolane-3,4-diol, a compound of formula (2) as a white solid.

B. Preparation of a Compound of Formula (2), varying $R^1$ and X

Similarly, following the procedure of 1A above, but replacing methylamine by propylamine and 3-iodobenzylamine, the following compounds of formula (2) were prepared: (4S,2R,3R,5R)-2-[2-chloro-6-(propylamino)purin-9-yl]-5-(hydroxymethyl)oxolane-3,4-diol; and (4S,2R,3R,5R)-2-[2-chloro-6-(3-iodobenzylamino)purin-9-yl]-5-(hydroxymethyl)oxolane-3,4-diol.

C. Preparation of a Compound of Formula (2), varying $R^1$ and X

Similarly, following the procedure of 1A above, but replacing methylamine by other compounds of formula $R^1XNH_2$, the following compounds of formula (2) are prepared:

(45,2R,3R,5R)-2-[2-chloro-6-(ethylamino)purin-9-yl]-5-(hydroxymethyl)oxolane-3,4-diol;
(45,2R,3R,5R)-2-[2-chloro-6-(n-propylamino)purin-9-yl]-5-(hydroxymethyl)oxolane-3,4-diol;
(4S,2R,3R,5R)-2-[2-chloro-6-(cyclopropylamino)purin-9-yl]-5-(hydroxymethyl)oxolane-3,4-diol;
(4S,2R,3R,5R)-2-[2-chloio-6-(cyclopropylmethylamino)purin-9-yl]-5-(hydroxymethyl)oxolane-3,4-diol;
(4S,2R,3R,5R)-2-[2-chloro-6-(cyclopentylamino)purin-9-yl]-5-(hydroxymethyl)oxolane-3,4-diol;
(45,2R,3R,5R)-2-[2-chloro-6-(anilinopurin-9-yl)]-5-(hydroxymethyl)oxolane-3,4-diol;
(4S,2R,3R,5R)-2-[2-chloro-6-(4-chlorobenzylamino)purin-9-yl]-5-(hydroxymethyl)oxolane-3,4-diol;
(4S,2R,3R,5R)-2-[2-chloro-6-(benzylamino)purin-9-yl]-5-(hydroxymethyl)oxolane-3,4-diol;
(4S,2R,3R,5R)-2-[2-chloro-6-(2-fluoxobenzylamino)purin-9-yl]-5-(hydroxymethyl)oxolane-3,4-diol;
(4S,2R,3R,5R)-2-[2-chloro-6-(pyrid-2-ylamino)purin-9-yl]-5-(hydroxymethyl)oxolane-3,4-diol; and
(4S,2R,3R,5R)-2-[2-chloro-6-(pyrrol-3-ylamino)purin-9-yl]-5-(hydroxymethyl)oxolane-3,4-diol.

D. Preparation of a Compound of Formula (2), varying $R^1$ and X

Similarly, following the procedure of 1A above, but replacing methylamine by other compounds of formula $R^1XNH_2$, other compounds of formula (2) are prepared.

EXAMPLE 2

Preparation of a Compound of Formula (3)

A. Preparation of a Compound of Formula (3) where $R^1$ is Methyl and X is a Covalent Bond

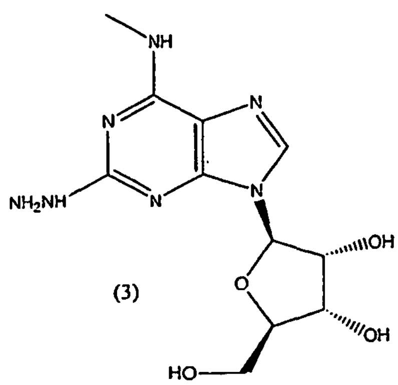

(3)

(4S,2R,3R,5R)-2-[2-chloro-6-(methylamino)purin-9-yl]-5-(hydroxymethyl)oxolane-3,4-diol, a compound of formula (2) (0.5 mmol), was suspended in hydrazine hydrate (5 mL), and the mixture was allowed to stir at room temperature for 24 hours. The hydrazine was removed under reduced pressure and the residue triturated with ether and filtered, to afford (4S,2R,3R,5R)-2-[2-hydrazino-6-(methylamino)purin-9-yl]-5-(hydroxymethyl)oxolane-3,4-diol, a compound of formula (3), as a white solid.

B. Preparation of a Compound of Formula (3), varying $R^1$ and X

Similarly, following the procedure of 2A above, but replacing 2-(2-chloro-6-methylaminopurin-9-yl)-5-hydroxymethyltetrahydrofuran-3,4-diol by the propylamino and 3-iodobenzylamino anaolgs of formula (2), the following compounds of formula (3) were prepared:

(4S,2R,3R,5R)-2-[2-hydrazino-6-(n-propylamino)purin-9-yl]-5-(hydroxymethyl)oxolane-3,4-diol; and
(4S,2R,3R,5R)-2-[2-hydrazino-6-(3-iodobenzylamino)purin-9-yl]-5-(hydroxymethyl)oxolane-3,4-diol.

C. Preparation of a Compound of Formula (3), varying $R^1$ and X

Similarly, following the procedure of 2A above, but replacing 2-(2-chloro-6-methylaminopurin-9-yl)-5-hydroxymethyltetrahydrofuran-3,4-diol by other compounds of formula (2), the following compounds of formula (3) are prepared:

(4S,2R,3R,5R)-2-[2-hydrazino-6-(ethylamino)purin-9-yl]-5-(hydroxymethyl)oxolane-3,4-diol;
(4S,2R,3R,5R)-2-[2-hydrazino-6-(cyclopropylamino)purin-9-yl]-5-(hydroxymethyl)oxolane-3,4-diol;
(45,2R,3R,5R)-2-[2-hydrazino-6-(cyclopropylmethylamino)purin-9-yl]-5-(hydroxymethyl)oxolane-3,4-diol;
(4S,2R,3R,5R)-2-[2-hydrazino-6-(cyclopentylamino)purin-9-yl]-5-(hydroxymethyl)oxolane-3,4-diol;
(4S,2R,3R,5R)-2-[2-hydrazino-6-(anilinopurin-9-yl)]-5-(hydroxymethyl)oxolane-3,4-diol;
(4S,2R,3R,5R)-2-[2-hydrazino-6-(4-chlorobenzylamino)purin-9-yl]-5-(hydroxymethyl)oxolane-3,4-diol;
(4S,2R,3R,5R)-2-[2-hydrazino-6-(benzylamino)purin-9-yl]-5-(hydroxymethyl)oxolane-3,4-diol;
(4S,2R,3R,5R)-2-[2-hydrazino-6-(2-fluorobenzylamino)purin-9-yl]-5-(hydroxymethyl)oxolane-3,4-diol;
(4S,2R,3R,5R)-2-[2-hydrazino-6-(pyrid-2-ylamino)purin-9-yl]-5-(hydroxymethyl)oxolane-3,4-diol; and
(4S,2R,3R,5R)-2-[2-hydrazino-6-(pyrrol-3-ylamino)purin-9-yl]-5-(hydroxymethyl)oxolane-3,4-diol.

D. Preparation of a Compound of Formula (3), varying $R^1$ and X

Similarly, following the procedure of 2A above, but replacing 2-(2-chloro-6-methylaminopurin-9-yl)-5-hydroxymethyltetrahydrofuran-3,4-diol by other compounds of formula (2), other compounds of formula (3) are prepared.

EXAMPLE 3

Preparation of a Compound of Formula I

A. Preparation of a Compound of Formula I where $R^1$ is Methyl, $R^2$ is 4-(4-Methoxyphenyl)pyrazol-1-yl, and X is a Covalent Bond

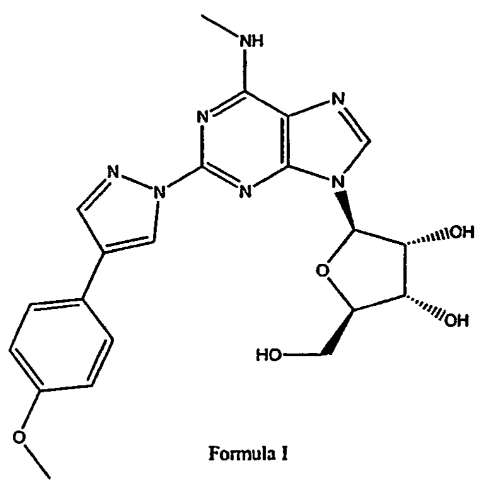

Formula I

(4S,2R,3R,5R)-2-[2-hydrazino-6-(methylamino)purin-9-yl]-5-(hydroxymethyl)oxolane-3,4-diol (0.5 mmol) was suspended in 3 mL of ethanol and to the suspension was added 2-(4-methoxyphenyl)malonaldehyde, a compound of formula (4). The mixture was heated at reflux for 5 hours, and the precipitate thus formed was collected by filtration, and washed with ethanol and ether to afford (4S,2R,3R,5R)-5-(hydroxymethyl)-2-{2-[4-(4-methoxyphenyl)pyrazolyl]-6-(methylamino)purin-9-yl}oxolane-3,4-diol, a compound of Formula I, Ms, 455.43 (M+1).

B. Preparation of a Compound of Formula I, varying $R^1$, $R^2$, and X

Similarly, following the procedure of 3A above, but optionally replacing 2-(2-hydrazino-6-methylaminopurin-9-yl)-5-hydroxymethyltetrahydrofuran-3,4-diol with other compounds of formula (3), and optionally replacing 2-(4-methoxyphenyl)malonaldehyde with other compounds of formula (4), the following compounds of Formula I were prepared:

(4S,2R,3R,5R)-5-(hydroxymethyl)-2-{2-[4-(4-ethoxycarbonyl)pyrazolyl]-6-(methylainino)purin-9-yl}oxolane-3,4-diol;
(4S,2R,3R,5R)-5-(hydroxymethyl)-2-{2-[4-(4-ethoxycarbonyl)pyrazolyl]-6-(n-propylamino)purin-9-yl}oxolane-3,4-diol;
(4S,2R,3R,5R)-5-(hydroxymethyl)-2-{2-[4-(4-ethoxycarbonyl)pyrazolyl]-6-(3-iodobenzylamino)purin-9-yl}oxolane-3,4-diol;
(4S,2R,3R,5R)-5-(hydroxymethyl)-2-{3-[4-(3-ethoxycarbonyl)pyrazolyl]-6-(methylamino)purin-9-yl}oxolane-3,4-diol;
(4S,2R,3R,5R)-5-(hydroxymethyl)-2-{2-[4-(4-ethoxycarbonyl)pyrazolyl]-6-(methylamino)purin-9-yl}oxolane-3,4-diol;
(4S,2R,3R,5R)-5-(hydroxymethyl)-2-{2-[4-(3-ethoxycarbonyl)pyrazolyl]-6-(3-iodobenzylamino)purin-9-yl}oxolane-3,4-diol;
(45,2R,3R,5R)-5-(hydroxymethyl)-2-{2-[4-(amido)pyrazolyl]-6-(methylamino)purin-9-yl}oxolane-3,4-diol;
(4S,2R,3R,5R)-5-(hydroxymethyl)-2-{2-[4-(methylamido)pyrazolyl]-6-(n-propylamino)purin-9-yl) oxolane-3,4-diol;
(4S,2R,3R,5R)-5-(hydroxymethyl)-2-{2-[4-(ethylamido)pyrazolyl]-6-(3-iodobenzylamino)purin-9-yl}oxolane-3,4-diol;
(4S,2R,3R,5R)-5-(hydroxymethyl)-2-{2-[4-(propylamido)pyrazolyl]-6-(3-iodobenzylamino)purin-9-yl}oxolane-3,4-diol;
(4S,2R,3R,5R)-5-(hydroxymethyl)-2-{2-[4-(cyclopentylamido)pyrazolyl]-6-(3-iodobenzylamino)purin-9-yl}oxolane-3,4-diol;
(4S,2R,3R,5R)-5-(hydroxymethyl)-2-{2-[4-(ethylamido)pyrazolyl]-6-(3-iodobenzylamino)purin-9-yl}oxolane-3,4-diol;
(4S,2R,3R,5R)-5-(hydroxymethyl)-2-{2-[4-(4-methylphenyl)pyrazolyl]-6-(methylamino)purin-9-yl}oxolane-3,4-diol;
(4S,2R,3R,5R)-5-(hydroxymethyl)-2-{2-[4-(4-methylphenyl)pyrazolyl]-6-(cyclopentylamino)purin-9-yl}oxolane-3,4-diol;
(4S,2R,3R,5R)-5-(hydroxymethyl)-2-{2-[4-(4-methoxyphenyl)pyrazolyl]-6-(n-propylamino)purin-9-yl}oxolme-3,4-diol;
(4S,2R,3R,5R)-5-(hydroxymethyl)-2-{2-[4-(4-methoxyphenyl)pyrazolyl]-6-(3-iodobenzylamino)purin-9-yl}oxolane-3,4-diol;
(4S,2R,3R,5R)-5-(hydroxymethyl)-2-{2-[4-(4-chlorophenyl)pyrazolyl]-6-(3-iodobenzylamino)purin-9-yl}oxolane-3,4-diol;
(4S,2R,3R,5R)-5-(hydroxymethyl)-2-[4-(pyrimidin-5-yl)pyrazolyl]-6-(methylamino)purin-9-yl}oxolane-3,4-diol;
(4S,2R,3R,5R)-5-(hydroxymethyl)-2-[4-(pyrimidin-5-yl)pyrazolyl]-6-(3-iodobenzylamino)purin-9-yl}oxolane-3,4-diol;
(4S,2R,3R,5R)-5-(hydroxymethyl)-2-[4-(pyridin-2-yl)pyrazolyl]-6-(n-propylamino)purin-9-yl}oxolane-3,4-diol;
(4S,2R,3R,5R)-5-(hydroxymethyl)-2-[4-(pyridin-2-yl)pyrazolyl]-6-(3-iodobenzylamino)purin-9-yl}oxolane-3,4-diol;
(4S,2R,3R,5R)-5-(hydroxymethyl)-2-[4-(pyridin-4-yl)pyrazolyl]-6-(methylamino)purin-9-yl}oxolane-3,4-diol;
(4S,2R,3R,5R)-5-(hydroxymethyl)-2-[4-(pyridin-4-yl)pyrazolyl]-6-(3-iodobenzylamino)purin-9-yl}oxolane-3,4-diol;
(4S,2R,3R,5R)-5-(hydroxymethyl)-2-[4-(pyridin-2-yl)pyrazolyl]-6-(methylamino)purin-9-yl}oxolane-3,4-diol;
(4S,2R,3R,5R)-5-(hydroxymethyl)-2-[4-(benzoxazol-2-yl)pyrazolyl]-6-(methylamino)purin-9-yl}oxolane-3,4-diol;
(4S,2R,3R,5R)-5-(hydroxymethyl)-2-[4-(benzoxazol-2-yl)pyrazolyl]-6-(3-iodobenzylamino)purin-9-yl}oxolane-3,4-diol;
(4S,2R,3R,5R)-5-(hydroxymethyl)-2-[4-(pyridin-2-yl)pyrazolyl]-6-(methylamino)purin-9-yl}oxolane-3,4-diol;
(4S,2R,3R,5R)-5-(hydroxymethyl)-2-[4-(quinolin-2-yl)pyrazolyl]-6-(3-methylamino)purin-9-yl}oxolane-3,4-diol;
(4S,2R,3R,5R)-5-(hydroxymethyl)-2-[4-(isoquinolin-1-yl)pyrazolyl]-6-(3-iodobenzylamino)purin-9-yl}oxolane-3,4-diol;
(4S,2R,3R,5R)-5-(hydroxymethyl)-2-{2-[3,5-dimethylpyrazolyl]-6-(methylamino)purin-9-yl}oxolane-3,4-diol;
(4S,2R,3R,5R)-5-(hydroxymethyl)-2-{2-[4-n-butyl-3,5-dimethylpyrazolyl]-6-(methylamino)purin-9-yl}oxolane-3,4-diol; and
(4S,2R,3R,5R)-5-(hydroxymethyl)-2-{2-[4-n-propyl-3,5-dimethylpyrazolyl]-6-(methylamino)purin-9-yl}oxolane-3,4-diol.

C. Preparation of a Compound of Formula I, varying $R^1$, $R^2$, and X

Similarly, following the procedure of 3A above, but optionally replacing 2-(2-hydrazino-6-methylaminopurin-9-yl)-5-hydroxymethyltetrahydrofuran-3,4-diol with other compounds of formula (3), and optionally replacing 2-(4-methoxyphenyl)malonaldehyde with other compounds of formula (4), the following compounds of Formula I are prepared:

(4S,2R,3R,5R)-5-(hydroxymethyl)-2-{2-[4-(4-methoxyphenyl)pyrazolyl]-6-(ethylamino)purin-9-yl}oxolane-3,4-diol;
(45,2R,3R,5R)-5-(hydroxymethyl)-2-{2-[4-ethoxycarbonyl)pyrazolyl]-6-(ethylamino)purin-9-yl}oxolane-3,4-diol;
(4S,2R,3R,5R)-5-(hydroxymethyl)-2-{2-[4-(4-methoxyphenyl)pyrazolyl]-6-(n-propylamino)purin-9-yl}oxolane-3,4-diol;
(4S,2R,3R,5R)-5-(hydroxymethyl)-2-{2-[4-(4-methoxyphenyl)pyrazolyl]-6-(cyclopropylamino)purin-9-yl}oxolane-3,4-diol;
(4S,2R,3R,5R)-5-(hydroxymethyl)-2-{2-[4-(4-methoxyphenyl)pyrazolyl]-6-(cyclopropylmethylamino)purin-9-yl}oxolane-3,4-diol;
(4S,2R,3R,5R)-5-(hydroxymethyl)-2-{2-[4-propylamido)pyrazolyl]-6-(cyclopropylamino)purin-9-yl}oxolane-3,4-diol;
(4S,2R,3R,5R)-5-(hydroxymethyl)-2-{2-[4-(4-methoxyphenyl)pyrazolyl]-6-(anilinopurin-9-yl}oxolane-3,4-diol;
(4S,2R,3R,5R)-5-(hydroxymethyl)-2-{2-[pyridin-4-yll)pyrazolyl]-6-(3-iodobenzylamino)purin-9-yl}oxolane-3,4-diol;
(4S,2R,3R,5R)-5-(hydroxymethyl)-2-{2-[4-(4-methoxyphenyl)pyrazolyl]-6-(benzylamino)purin-9-yl}oxolane-3,4-diol;
(45,2R,3R,5R)-5-(hydroxymethyl)-2-{2-[4-(4-methoxyphenyl)pyrazolyl]-6-(2-fluorobenzylamino)purin-9-yl}oxolane-3,4-diol;
(4S,2R,3R,5R)-5-(hydroxymethyl)-2-{2-[4-(4-methoxyphenyl)pyrazolyl]-6-(pyrid-2-ylamino)purin-9-yl}oxolane-3,4-diol; and
(4S,2R,3R,5R)-5-(hydroxymethyl)-2-{2-[4-(4-methoxyphenyl)pyrazolyl]-6-(pyrrol-3-ylmethylamino)purin-9-yl}oxolane-3,4-diol.

D. Preparation of a Compound of Formula I, varying $R^1$, $R^2$, and X

Similarly, following the procedure of 3A above, but optionally replacing 2-(2-hydrazino-6-methylaminopurin-9-yl)-5-hydroxymethyltetrahydrofuran-3,4-diol with other compounds of formula (3), and optionally replacing 2-(4-methoxyphenyl)malonaldehyde with other compounds of formula (4), other compounds of Formula I are prepared:

EXAMPLE 4

Preparation of a Compound of Formula (12)

A. Preparation of a Compound of Formula (12) where $R^1$ is Methyl

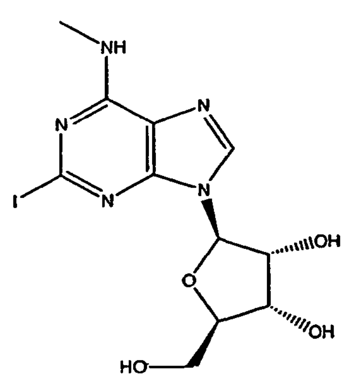

A mixture of 40% aqueous methylamine (1 mL) and 2-iodoadenosine (100 mg) in methanol (2 mL) was stirred at room temperature for 12 hours. The precipitate was filtered off, washed with ether and dried under vacuum to afford

2-iodo-6-methylamino adenosine, a compound of formula (11).

B. Preparation of a Compound of Formula (12), varying $R^1$

Similarly, following the procedure of 4A above, but replacing methylamine with other amines of formula $R^1NH_2$, the following compounds of formula (11) were prepared:

2-iodo-6-n-propylamino adenosine; and
2-iodo-6-(3-iodobenzyl)amino adenosine.

C. Preparation of a Compound of Formula (12), varying $R^1$

Similarly, following the procedure of 4A above, but replacing methylamine with other amines of formula HNRR, the following compounds of formula (11) are prepared:

2-iodo-6-ethylamino adenosine;
2-iodo-6-isopropylamino adenosine;
2-iodo-6-n-hexylamino adenosine;
2-iodo-6-cyclopropylamino adenosine;
2-iodo-6-cyclopentylamino adenosine;
2-iodo-6-(3-hydroxycyclopentyl)amino adenosine;
2-iodo-6-cyclopentylmethylamino adenosine;
2-iodo-6-phenylamino adenosine;
2-iodo-6-benzylamino adenosine;
2-iodo-6-(4-methoxybenzyl)amino adenosine;
2-iodo-6-(4-fluorobenzyl)amino adenosine;
2-iodo-6-(pyridy-3-yl)amino adenosine; and
2-iodo-6-(furan-2-yl)amino adenosine.

D. Preparation of a Compound of Formula (12), varying $R^1$

Similarly, following the procedure of 4A above, but replacing methylamine with other amines of formula HNRR, other compounds of formula (11) are prepared.

EXAMPLE 5

Preparation of a Compound of Formula I

A. Preparation of a Compound of Formula I where $R^1$ is Methyl, $R^2$ is 3-phenoxypropyn-1-yl, and $R^8$ is Phenyl

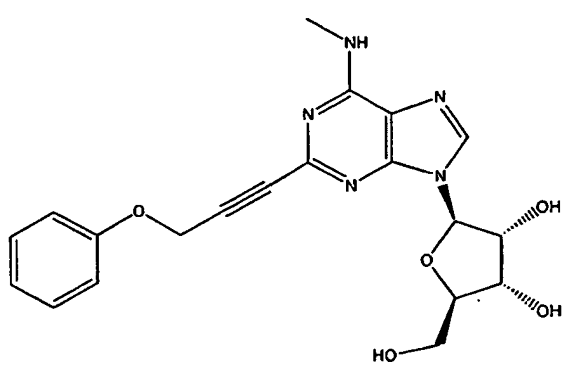

To a solution of 2-iodo-6-methylamino adenosine (50 mg) and prop-2-ynyloxybenzene (0.022 mL, 0.16 mmol) in N, N-dimethylformamide (1 ml) and triethylamine (0.021 mL, 0.16 mmol) at 23 C was added copper iodide (5mg, 0.026 mmol) and dichlorobis-(triphenylphosphine)palladium(II) (22 mg, 0.031 mmol) catalyst. After being stirred in a

sealed reaction-vial at 80°C for 6 hours, the reaction was concentrated *in vacuo*, and the residue purified by preparatory thin layer chromatography (methylene chloride: methanol 9:1) to afford (4S,2R,3R,5R)-5-(hydroxymethyl)-2-[6-(methylamino)-2-(3-phenoxyprop-1-ynyl)purin-9-yl]oxolane-3,4-diol, a compound of Formula I. MS: 412.1 (M+1).

B. Preparation of a Compound of Formula I, varying $R^2$

Similarly, following the procedure of 5A above, but replacing prop-2-ynyloxybenzene with other compounds of formula (7), the following compounds of Formula I were prepared:

(45,2R,3R,5R)-5-(hydroxymethyl)-2-[2-(3-hydroxy-3-phenylprop-1-ynyl)-6-(methylamino)purin-9-yl]oxolane-3,4-diol;
(4S,2R,3R,5R)-2-{2-[3-(4-chlorophenoxy)prop-1-ynyl]-6-(methylamino)purin-9-yl}-5-(hydroxymethyl)oxolane-3,4-diol; and
(4S,2R,3R,5R)-2-{2-[3-(2-methoxyphenoxy)prop-1-ynyl]-6-(methylamino)purin-9-yl}-5-(hydroxymethyl)oxolane-3,4-diol.

C. Preparation of a Compound of Formula I, varying $R^1$, $R^2$, and X

Similarly, following the procedure of 5A above, but replacing prop-2-ynyloxybenzene with other compounds of formula (7), the following compounds of Formula I are prepared:

(4S,2R,3R,5R)-5-(hydroxymethyl)-2-[6-(ethylamino)-2-(3-phenoxyprop-1-ynyl)purin-9-yl]oxolane-3,4-diol;
(45,2R,3R,5R)-5-(hydroxymethyl)-2-[6-(n-propylamino)-2-(3-phenoxyprop-1-ynyl)purin-9-yl]oxolane-3,4-diol;
(4S,2R,3R,5R)-5-(hydroxymethyl)-2-[6-(isopropylamino)-2-(3-phenoxyprop-1-ynyl)purin-9-yl]oxolane-3,4-diol;
(45,2R,3R,5R)-5-(hydroxymethyl)-2-[6-(n-hexylamino)-2-(3-phenoxyprop-1-ynyl)purin-9-yl]oxolane-3,4-diol;
(45,2R,3R,5R)-5-(hydroxymethyl)-2-[6-(cyclopropylamino)-2-(3-phenoxyprop-1-ynyl)purin-9-yl]oxolane-3,4-diol;
(4S,2R,3R,5R)-5-(hydroxymethyl)-2-[6-(cyclopentylamino)-2-(3-phenoxyprop-1-ynyl)purin-9-yl]oxolane-3,4-diol;
(4S,2R,3R,5R)-5-(hydroxymethyl)-2-[6-(3-hydroxycyclopentylamino)-2-(3-phenoxyprop-1-ynyl)purin-9-yl]oxolane-3,4-diol;
(4S,2R,3R,5R)-5-(hydroxymethyl)-2-[6-(cyclopentylmethylamino)-2-(3-phenoxyprop-1-ynyl)purin-9-yl]oxolane-3,4-diol;
(4S,2R,3R,5R)-5-(hydroxymethyl)-2-[6-(phenylamino)-2-(3-phenoxyprop-1-ynyl)purin-9-yl]oxolane-3,4-diol;
(4S,2R,3R,5R)-5-(hydroxymethyl)-2-[6-(benzylamino)-2-(3-phenoxyprop-1-ynyl)purin-9-yl]oxolane-3,4-diol;
(4S,2R,3R,5R)-5-(hydroxymethyl)-2-[6-(4-methoxybenzylamino)-2-(3-phenoxyprop-1-ynyl)purin-9-yl]oxolane-3,4-diol;
(4S,2R,3R,5R)-5-(hydroxymethyl)-2-[6-(4-fluorobenzylamino)-2-(3-phenoxyprop-1-ynyl)purin-9-yl]oxolane-3,4-diol;
(4S,2R,3R,5R)-5-(hydroxymethyl)-2-[6-(pyrid-3-ylamino)-2-(3-phenoxyprop-1-ynyl)purin-9-yl]oxolane-3,4-diol; and
(4S,2R,3R,5R)-5-(hydroxymethyl)-2-[6-(furan-2-ylamino)-2-(3-phenoxyprop-1-ynyl)purin-9-yl]oxolane-3,4-diol.

D. Preparation of a Compound of Formula I, varying $R^1$, $R^2$, and X

Similarly, following the procedure of 5A above, but replacing prop-2-ynyloxybenzene with other compounds of formula (7), other compounds of Formula I are prepared.

EXAMPLE 6

Following the procedures shown in Reaction Scheme II above, as detailed in U.S. Patent No. 6,214,807, the following compounds of Formula I in which $R^2$ is an optionally substituted C-pyrazole were made:

(1-{9-[(4S,2R,3R,5R)-3,4-dihydroxy-5-(hydroxymethyl)oxolan-2-yl]-6-(methylamino)purin-2-yl}pyrazol-4-yl)-N-(4-chlorophenyl)carboxamide;
(4S,2R,3R,5R)-5-(hydroxymethyl)-2-{2-[1-benzylpyrazol-4-yl]-6-(methylamino)purin-9-yl}oxolane-3,4-diol;
(4S,2R,3R,5R)-5-(hydroxymethyl)-2-{2-[1-benzylpyrazol-4-yl]-6-(n-propylamino)purin-9-yl}oxolane-3,4-diol;
(4S,2R,3R,5R)-5-(hydroxymethyl)-2-{2-[1-benzylpyrazol-4-yl]-6-(3-iodophenylamino)purin-9-yl}oxolane-3,4-diol;

The following examples illustrate the preparation of representative pharmaceutical formulations containing a

compound of Formula I, such as those prepared in accordance with Example 1.

EXAMPLE 7

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules.

EXAMPLE 8

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets.

EXAMPLE 9

A dry powder inhaler formulation is prepared containing the following components:

| Ingredient | Weight % |
|---|---|
| Active Ingredient | 5 |
| Lactose | 95 |

The active ingredient is mixed with the lactose and the mixture is added to a dry powder inhaling appliance.

EXAMPLE 10

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone | |
| (as 10% solution in sterile water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50 °C to 60 °C and passed through a 16 mesh U.S. sieve. The sodium

carboxymethyl starch, magnesium stearate, and talc, previously passed through a No. 30 mesh U.S. sieve, are then added to the granules which, after mixing, are compressed on a tablet machine to yield tablets each weighing 120 mg.

EXAMPLE 11

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

EXAMPLE 12

Suspensions, each containing 50 mg of active ingredient per 5.0 mL dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

EXAMPLE 13

A subcutaneous formulation may be prepared as follows:

| Ingredient | Quantity |
|---|---|
| Active Ingredient | 5.0 mg |
| Corn Oil | 1.0 mL |

EXAMPLE 14

An injectable preparation is prepared having the following composition:

| Ingredients | Amount |
|---|---|
| Active ingredient | 2.0 mg/ml |
| Mannitol, USP | 50 mg/ml |
| Gluconic acid, USP | q.s. (pH 5-6) |
| water (distilled, sterile) | q.s. to 1.0 ml |
| Nitrogen Gas, NF | q.s. |

EXAMPLE 15

A topical preparation is prepared having the following composition:

| Ingredients | grams |
|---|---|
| Active ingredient | 0.2-10 |
| Span 60 | 2.0 |
| Tween 60 | 2.0 |
| Mineral oil | 5.0 |
| Petrolatum | 0.10 |
| Methyl paraben | 0.15 |
| Propyl paraben | 0.05 |
| BHA (butylated hydroxy anisole) | 0.01 |
| Water | q.s. to100 |

All of the above ingredients, except water, are combined and heated to 60) C with stirring. A sufficient quantity of water at 60) C is then added with vigorous stirring to emulsify the ingredients, and water then added q.s. 100 g.

EXAMPLE 16

Sustained Release Composition

| Ingredient | Weight Range (%) | Preferred Range (%) | Most Preferred |
|---|---|---|---|
| Active ingredient | 50-95 | 70-90 | 75 |
| Microcrystalline cellulose (filler) | 1-35 | 5-15 | 10.6 |
| Methacrylic acid copolymer | 1-35 | 5-12.5 | 10.0 |
| Sodium hydroxide | 0.1-1.0 | 0.2-0.6 | 0.4 |
| Hydroxypropyl methylcellulose | 0.5-5.0 | 1-3 | 2.0 |
| Magnesium stearate | 0.5-5.0 | 1-3 | 2.0 |

The sustained release formulations of this invention are prepared as follows: compound and pH-dependent binder and any optional excipients are intimately mixed(dry-blended). The dry-blended mixture is then granulated in the presence of an aqueous solution of a strong base which is sprayed into the blended powder. The granulate is dried, screened, mixed with optional lubricants (such as talc or magnesium stearate), and compressed into tablets. Preferred aqueous solutions of strong bases are solutions of alkali metal hydroxides, such as sodium or potassium hydroxide, preferably sodium hydroxide, in water (optionally containing up to 25% of water-miscible solvents such as lower alcohols).

The resulting tablets may be coated with an optional film-forming agent, for identification, taste-masking purposes and to improve ease of swallowing. The film forming agent will typically be present in an amount ranging from between 2% and 4% of the tablet weight. Suitable film-forming agents are well known to the art and include hydroxypropyl. methylcellulose, cationic methacrylate copolymers (dimethylaminoethyl methacrylate/methyl-butyl methacrylate copolymers - Eudragit® E - Röhm. Pharma), and the like. These film-forming agents may optionally contain colorants, plasticizers, and other supplemental ingredients.

The compressed tablets preferably have a hardness sufficient to withstand δ Kp compression. The tablet size will depend primarily upon the amount of compound in the tablet. The tablets will include from 300 to 1100 mg of compound free base. Preferably, the tablets will include amounts of compound free base ranging from 400-600 mg, 650-850 mg, and 900-1100 mg.

In order to influence the dissolution rate, the time during which the compound containing powder is wet mixed is controlled. Preferably the total powder mix time, i.e. the time during which the powder is exposed to sodium hydroxide solution, will range from 1 to 10 minutes and preferably from 2 to 5 minutes. Following granulation, the particles are removed from the granulator and placed in a fluid bed dryer for drying at about 60°C.

Abbreviations

Gpp(NH)p: 5'-guanylyl-imididodiphosphate
R-PIA: phenylisopropyladenosine
TEM buffer: Buffer containing 50mM Tris, 1 mM EDTA and 10mM MgCl2

Reagents

Adenosine deaminase is purchased from Boehringer Mannheim Biochemicals Indianapolis, IN). R-PIA, DMSO and rolipram are obtained from Sigma-RBI (Natick, MA).

Binding Assays

Cell Culture

CHO cells (Chinese hamster ovary cells), stably transfected with human adenosine $A_3$ receptors, are grown as monolayers in petri dishes using Dulbecco's Modified Eagle's Medium (DMEM) containing 2.5 $\mu g\ ml^{-1}$ amphotericin B, 100 U $ml^{-1}$ penicillin G, 0.1 mg $ml^{-1}$ streptomycin sulfate and 5% fetal bovine serum in a humidified atmosphere of 95% air and 5% $CO_2$. Cells are subcultured twice weekly by dispersion in Hank's Balanced Salt Solution (HBSS) without the divalent cations and containing 1 mM EDTA. The cells are then seeded in growth medium at a density of 1.2 x $10^5$ cells per plate and experiments are performed 4 days later at approximately one day preconfluence.

Membrane Preparations

Attached cells are washed twice with HBSS (2 x 10 ml), scraped free of the plate with the aid of a rubber policeman in 5 ml of 50 mM Tris-HCl buffer pH 7.4 at 4 °C and the suspension homogenized for 10 s. The suspension is then centrifuged at 27,000 x g for 10 min. The pellet is resuspended in homogenization buffer by vortexing and centrifuged as described above. The final pellet is resuspended in 1 vol of 50 mM Tris-HCl buffer pH 7.4 containing 5 mM $MgCl_2$ for binding assays. For the [$^{35}$S]GTPγS binding assay the final pellet is resuspended in 50 mM Tris-HCl pH 7.4 containing 5 mM $MgCl_2$, 100 mM NaCl and 1 mM dithiothreitol. This membrane suspension is then placed in liquid nitrogen for 10 min, thawed and used for assays. The protein content is determined with a Bradford™ Assay Kit using bovine serum albumin as standard.

Competitive Binding Assay

Compounds of Formula I are assayed to determine their affinity for the human adenosine $A_3$ receptors in membranes extracted from CHO cells. Briefly, 0.2 mg of cell membranes are incubated with adenosine deaminase and 50 mM Tris buffer (pH = 7.4) with constant mixing. 2 μL of serially diluted DMSO stock solution of the compounds of this invention at concentrations ranging from 0 to 100 μM to 10 nM. The control received 2 μL of DMSO alone, then [$^3$H] phenylisopropyladenosine ($^3$H PIA), an A3 receptor agonist, is disolved in Tris buffer (50 mM, pH of 7.4) and added to the cells (final concentration is 2 nM). After incubation at 23°C for 2hours, the solutions are filtered and the filter disks are placed in a scintillation cocktail. The displacement of [$^3$H] phenylisopropyladenosine by the competitive binding compositions of this invention was determined by scintillation counting.

Data generated from a displacement experiment are generally fitted by a sigmoidal curve termed the displacement curve, that is the percentage radiolabeled ligand specifically bound versus log [displacer] in M). The abscissa of the inflexion point of the curve gives the $IC_{50}$ value, the concentration of displacer that displaces or inhibits 50% of the radioactive ligand specifically bound. $IC_{50}$ is a measure of the inhibitor or affinity constant (Ki) of the displacer for the receptor. $IC_{50}$ and $K_i$ are linked as follows if the displacement is of the competitive type then

$$K_i=IC_{50}/(1+[C^*]/K_d^*$$

This is the Cheng- Prusoff equation (Biochem. Pharmacol, 22:3099 (1973)) where [C*] is the concentration of radioligand and $K_d$* is its dissociation constant.

EXAMPLE 17

**[$^{35}$S]GTPγS Binding Assays**

The ability of the adenosine $A_3$-agonists to stimulate [$^{35}$S] GTPγS binding is determined by a modification of the method described by Lorenzen et al. (1996 Mol. Pharmacol. 49:915). Briefly, membranes isolated from CHO cells (30-50 μg) are incubated in a volume of 0.1 ml containing 50 mM Tris-HCl buffer pH 7.4, 5 mM $MgCl_2$, 100 mM NaCl, 1 mM dithiothreitol, 0.2 units $ml^{-1}$ adenosine deaminase, 0.5% BSA, 1 mM EDTA, 10 mM GDP, and 0.3 nM [$^{35}$S]GTPγS. Various concentrations of PIA or the putative A3 agonists are added and the cells incubated for 90 min at 30°C. Nonspecific binding is determined by the addition of 10 μM GTPγS to some of the membrane suspensions. At the end of the incubation, each suspension is filtered and the retained radioactivity determined as described above.

EXAMPLE 18

cAMP Assay

The ability of the compounds to inhibit forskolin stimulated cAMP accumulation is determined by culturing $CHO_1$ cells in clear bottomed 96 well microtiter plates at concentrations between $10^4$ to $10^6$ cells per well in 40 ul of HBSS at 37°C (5% $CO_2$ and 95% humidity). CHO cells are incubated with various concentrations of the putative adenosine $A_3$ receptor agonists in the presence of rolipram (50uM), and 5 uM forskolin, for 10 min at 37°C. The cells are immediately lysed by treatment 5 ul of 10% dodecyltrimethylammonium bromide followed by shaking using microplate shaker.

The cAMP content of the supernatant is determined by modification of a radioimmunoassay method described by Harper and Brooker (1975. J. Cyclic nucleotide Res 1:207). Briefly, an aliquot of the supernatant (0.01 mL) is mixed with 0.04 mL of HBSS, 0.05 mL of 50 mmol/L sodium acetate buffer (pH 6.2) containing 10 mmol/L $CaCl_2$, [$^{125}$I] ScAMP-TME (12500 dpm), and 0.05 mL ofanti-cAMP antibody (1:2000 dilution with 0.1% bovine serum albumin in distilled water). The samples were then incubated at 4 °C for 16 hours. At the end of the incubation, 70 μL of a 50% (wt/vol) hydroxyapatite suspension is added to each tube. The suspensions were gently agitated and then incubated for 10 minutes at 4°C. Antibody-bound radioactivity adsorbed to hydroxyapatite is collected onto glass fiber filters by vacuum filtration using a Brandel cell harvester. Radioactivity retained by the filter is counted in a gamma counter. The results are expressed as the total [$I^{125}$I]ScAMP bound minus the amount of nonspecific ([$^{125}$I]ScAMP-TME bound (i. e. amount of [$I^{125}$I]ScAMP bound in the presence of 3 μmol/L unlabeled cAMP).

## Claims

**1.** A compound of the formula :

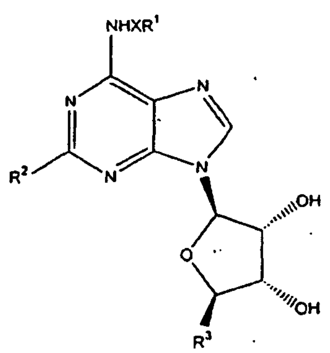

Formula I

wherein:

$R^1$ is optionally substituted lower alkyl, optionally substituted cycloalkyl, optionally substituted aryl, or optionally substituted heteroaryl;
X is a covalent bond or optionally substituted alkylene;

$R^2$ is $R^4$-Z-Y-C≡C- or optionally substituted pyrazolyl:

in which Y is optionally substituted alkylene, Z is oxygen, sulfur or -NH-, and $R^4$ is optionally substituted aryl or optionally substituted heteroaryl; and

$R^3$ is hydroxymethyl or -C(O)-$NR^5R^6$;

in which $R^5$ and $R^6$ are independently hydrogen or lower alkyl.

**2.** The compound of claim 1, wherein $R^2$ is optionally substituted pyrazol-1-yl.

**3.** The compound of claim 2, wherein $R^1$ is optionally substituted alkyl or optionally substituted aryl and $R^3$ is hydroxymethyl.

**4.** The compound of claim 3, wherein $R^2$ is pyrazol-1-yl substituted by optionally substituted lower alkyl, ester, aminocarbonyl, optionally substituted aryl, or optionally substituted heteroaryl.

**5.** The compound of claim 4, wherein pyrazol-1-yl is substituted by optionally substituted phenyl or optionally substituted benzyl.

**6.** The compound of claim 5, wherein $R^1$ is optionally substituted lower alkyl and X is a covalent bond.

**7.** The compound of claim 6, wherein $R^1$ is methyl and $R^2$ is 4-(4-methoxyphenyl)pyrazol-1-yl, namely (4S,2R,3R,5R)-5-(hydroxymethyl)-2-{2-[4-(4-methoxyphenyl)pyrazolyl]-6-(methylamino)purin-9-yl}oxolane-3,4-diol.

**8.** The compound of claim 6, wherein $R^1$ is n-propyl and $R^2$ is 4-(4-methoxyphenyl)pyrazol-1-yl, namely (4S,2R,3R,5R)-5-(hydroxymethyl)-2-{2-[4-(4-methoxyphenyl)pyrazolyl]-6-(n-propylamino)purin-9-yl}oxolane-3,4-diol.

**9.** The compound of claim 1, wherein $R^1$ is methyl and $R^2$ is 4-(4-chlorobenzylaminocarbonyl)pyrazol-1-yl, namely (1-{9-[(4S,2R,3R,5R)-3,4-dihydroxy-5-(hydroxymethyl)oxolan-2-yl]-6-(methylamino)purin-2-yl}pyrazol-4-yl)-N-(4-chlorobenzyl)carboxamide.

**10.** The compound of claim 1, wherein $R^1$ is methyl and $R^2$ is 4-(4-chlorophenylaminocarbonyl)pyrazol-1-yl, namely (1-{9-[(4S,2R,3R,5R)-3,4-dihydroxy-5-(hydroxymethyl)oxolan-2-yl]-6-(methylamino)purin-2-yl}pyrazol-4-yl)-N-(4-chlorophenyl)carboxamide.

**11.** The compound of claim 4, wherein $R^2$ is pyrazol-1-yl substituted by optionally substituted heteroaryl.

**12.** The compound of claim 11, wherein $R^1$ is n-propyl and $R^2$ is 4-(pyrid-2-yl)pyrazol-1-yl, namely (4S,2R,3R,5R)-5-(hydroxymethyl)-2-[4-(pyridin-2-yl)pyrazolyl]-6-(n-propylamino)purin-9-yl}oxolane-3,4-diol.

**13.** The compound of claim 5, wherein $R^1$ is optionally substituted aryl and X is alkylene.

**14.** The compound of claim 13, wherein X-$R^1$ is 3-iodobenzyl and $R^2$ is 4-(4-methoxyphenyl)pyrazol-1-yl, namely (4S,2R,3R,5R)-5-(hydroxymethyl)-2-{2-[4-(4-methoxyphenyl)pyrazolyl]-6-(3-iodobenzylamino)purin-9-yl}oxolane-3,4-diol.

**15.** The compound of claim 1, wherein $R^2$ is optionally substituted pyrazol-4-yl.

**16.** The compound of claim 15, wherein $R^1$ is optionally substituted alkyl or optionally substituted aryl, $R^3$ is hydroxymethyl, and X is a covalent bond.

**17.** The compound of claim 16, wherein $R^1$ is methyl, $R^2$ is 1-benzylpyrazol-4-yl, $R^3$ is hydroxymethyl, and X is a covalent bond, namely (4S,2R,3R,5R)-5-(hydroxymethyl)-2-{2-[1-benzylpyrazolyl]-6-(methylamino)purin-9-yl}oxolane-3,4-diol.

**18.** The compound of claim 16, wherein $R^1$ is n-propyl, $R^2$ is 1-benzylpyrazol-4-yl, $R^3$ is hydroxymethyl, and X is a covalent bond, namely (4S,2R,3R,5R)-5-(hydroxymethyl)-2-{2-[1-benzylpyrazolyl]-6-(n-propylamino)purin-9-yl}oxolane-3,4-diol.

**19.** The compound of claim 1, wherein $R^2$ is $R^4$-Z-Y-C≡C-.

**20.** The compound of claim 19, wherein $R^4$ is optionally substituted phenyl and Y is alkylene of 1-3 carbon atoms.

**21.** The compound of claim 20, wherein $R^4$ is phenyl optionally substituted by methoxy or chloro, and Y is methylene.

**22.** The compound of claim 21, wherein $R^1$ is optionally substituted alkyl, X is a covalent bond, and $R^3$ is hydroxymethyl.

**23.** The compound of claim 22, wherein $R^1$ is methyl, $R^4$ is phenyl and Z is oxygen, namely 2-hydroxymethyl-5-[6-methylamino-2-(3-phenoxypropyn-1-yl)purin-9-yl]-tetrahydrofuran-3,4-diol.

**24.** Use of a compound of any one of claims 1 to 23 for the preparation of a pharmaceutical composition for treating a disease state in a mammal that is alleviable by treatment with a $A_3$ adenosine receptor agonist.

**25.** The use of claim 24, wherein the disease state is cancer.

**26.** The use of claim 24, wherein the disease state is neutropenia.

**27.** A pharmaceutical composition comprising at least one pharmaceutically acceptable excipient and a therapeutically effective amount of a compound of any one of claims 1 to 23.

**28.** A process for the preparation of a compound of Formula I:

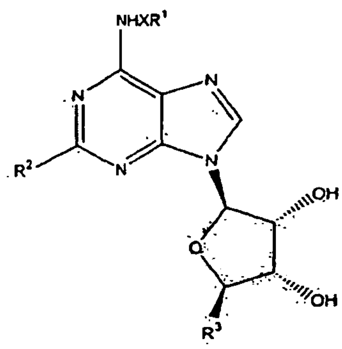

Formula I

in which
$R^1$ is optionally substituted lower alkyl, optionally substituted cycloalkyl, optionally substituted aryl, or optionally substituted heteroaryl;
X is a covalent bond or optionally substituted alkylene;
$R^2$ is optionally substituted pyrazol-1-yl; and
$R^3$ is hydroxymethyl or -C(O)-$NR^5R^6$;
in which $R^5$ and $R^6$ are independently hydrogen or lower alkyl,
comprising:

contacting a compound of the formula:

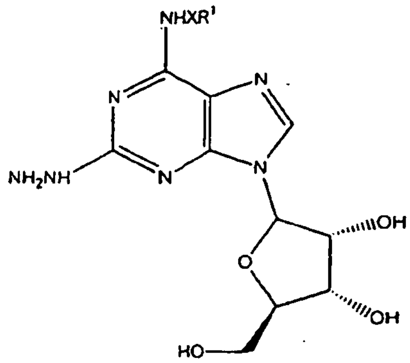

(3)

with a compound of formula:

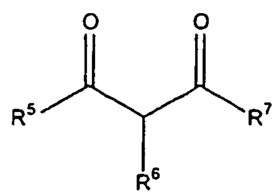

(4)

**29.** The process of claim 28, wherein the reaction is conducted in an inert solvent chosen from methanol, ethanol, n-propanot, isopropanol, and t-butanol.

**30.** A process for the preparation of a compound of Formula I:

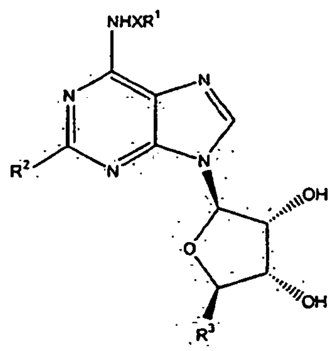

Formula I

in which
$R^1$ is optionally substituted lower alkyl, optionally substituted cycloalkyl, optionally substituted aryl, or optionally substituted heteroaryl;
X is a covalent bond or optionally substituted alkylene; and
$R^2$ is optionally substituted pyrazol-4-yl;
comprising:

contacting a compound of the formula:

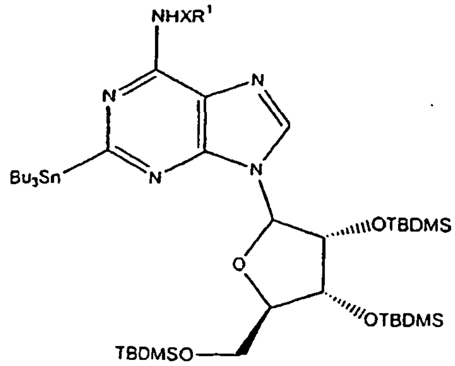

(8)

with a compound of the formula:

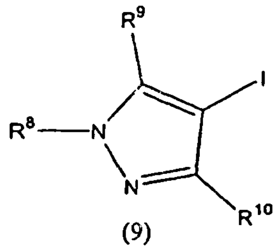

(9)

wherein $R^8$, $R^9$, and $R^{10}$ are independently hydrogen or alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl (an alkyl ester), arylthio, heteroaryl, heteroarylthio, heterocyclylthio, thiol, alkylthio, aryl, aryloxy, aralkyl, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, $-SO_2$-alkyl, $-SO_2$-aryl and $-SO_2$-heteroaryl;
in the presence of a palladium complex and a copper salt in an inert solvent, and contacting the product with a mild acid.

**31.** The process of claim 30, wherein the palladium complex is $Pd(PPh_3)_4$, the copper salt is CuI, the inert solvent is N,N-dimethylformamide, and the mild acid is ammonium fluoride.

**32.** A process for the preparation of a compound of claim 1, in which $R^2$ is $R^4$-Z-Y-C≡C-; comprising:

contacting in an inert solvent a compound of the formula:

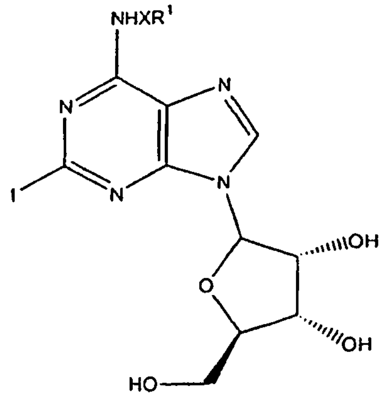

(12)

with a compound of the formula:

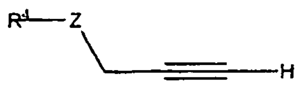

(13)

in the presence of a mild base, a copper salt and a palladium catalyst.

**33.** The process of claim 32, wherein the inert solvent is N,N-dimethylformamide, the base is triethylamine, the copper salt is copper iodide, and the palladium catalyst is dichlorobis(triphenylphosphine)palladium (II).

**Patentansprüche**

**1.** Verbindung der Formel:

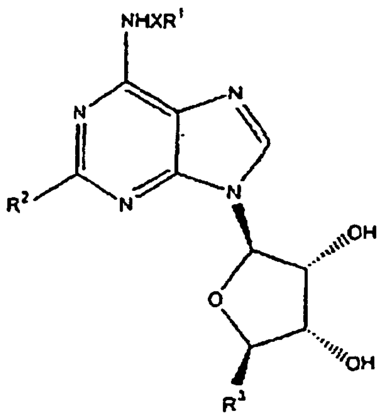

Formel I

worin:

$R^1$ eine gegebenenfalls substituierte Niederalkyl-, gegebenenfalls substituierte Cycloalkyl-, gegebenenfalls substituierte Aryl- oder gegebenenfalls substituierte Heteroarylgruppe ist,

X eine kovalente Bindung oder eine gegebenenfalls substituierte Alkylengruppe ist,
$R^2$ eine $R^4$-Z-Y-C≡C-- oder eine gegebenenfalls substituierte Pyrazolylgruppe ist,
in der Y eine gegebenenfalls substituierte Alkylengruppe ist, Z ein Sauerstoff-, Schwefelatom oder eine -NH--Gruppe ist und $R^4$ eine gegebenenfalls substituierte Aryl- oder gegebenenfalls substituierte Heteroarylgruppe ist, und
$R^3$ eine Hydroxymethyl- oder -C(O)-$NR^5R^6$-Gruppe ist,

in der $R^5$ und $R^6$ unabhängig ein Wasserstoffatom oder eine Niederalkylgruppe sind.

**2.** Verbindung nach Anspruch 1, wobei $R^2$ eine gegebenenfalls substituierte Pyrazol-1-yl-Gruppe ist.

**3.** Verbindung nach Anspruch 2, wobei $R^1$ eine gegebenenfalls substituierte Alkyl- oder gegebenenfalls substituierte Arylgruppe ist und $R^3$ eine Hydroxymethylgruppe ist.

**4.** Verbindung nach Anspruch 3, wobei $R^2$ eine Pyrazol-1-yl-Gruppe ist, die durch eine gegebenenfalls substituierte Niederalkyl-, Ester-, Aminocarbonyl-, gegebenenfalls substituierte Aryl- oder gegebenenfalls substituierte Heteroarylgruppe substituiert ist.

**5.** Verbindung nach Anspruch 4, wobei die Pyrazol-1-yl-Gruppe durch eine gegebenenfalls substituierte Phenyl- oder gegebenenfalls substituierte Benzylgruppe substituiert ist.

**6.** Verbindung nach Anspruch 5, wobei $R^1$ eine gegebenenfalls substituierte Niederalkylgruppe ist und X eine kovalente Bindung ist.

**7.** Verbindung nach Anspruch 6, wobei $R^1$ eine Methylgruppe ist und $R^2$ eine 4-(4-Methoxyphenyl)pyrazol-1-yl-Gruppe ist, nämlich (4S,2R,3R,5R)-5-(Hydroxymethyl)-2-{2-[4-(4-methoxyphenyl)pyrazolyl]-6-(methylamino)-purin-9-yl}oxolan-3,4-diol.

**8.** Verbindung nach Anspruch 6, wobei $R^1$ eine n-Propylgruppe ist und $R^2$ eine 4-(4-Methoxyphenyl)pyrazol-1-yl-Gruppe ist, nämlich (4S,2R,3R,5R)-5-(Hydroxymethyl)-2-{2-[4-(4-methoxyphenyl)pyrazolyl]-6-(n-propylamino)-purin-9-yl}oxolan-3,4-diol.

**9.** Verbindung nach Anspruch 1, wobei $R^1$ eine Methylgruppe ist und $R^2$ eine 4-(4-Chlorbenzylaminocarbonyl)pyrazol-1-yl-Gruppe ist, nämlich (1-{9-[(4S,2R,3R,5R)-3,4-Dihydroxy-5-(hydroxymethyl)oxolan-2-yl]-6-(methylamino)purin-2-yl}pyrazol-4-yl)-N-(4-chlorbenzyl)carboxamid.

**10.** Verbindung nach Anspruch 1, wobei $R^1$ eine Methylgruppe ist und $R^2$ eine 4-(4-Chlorphenylaminocarbonyl)pyrazol-1-yl-Gruppe ist, nämlich (1-{9-[(4S,2R,3R,5R)-3,4-Dihydroxy-5-(hydroxymethyl)oxolan-2-yl]-6-(methylamino)purin-2-yl}pyrazol-4-yl)-N-(4-chlorphenyl)carboxamid.

**11.** Verbindung nach Anspruch 4, wobei $R^2$ eine Pyrazol-1-yl-Gruppe ist, die durch eine gegebenenfalls substituierte Heteroarylgruppe substituiert ist.

**12.** Verbindung nach Anspruch 11, wobei $R^1$ eine n-Propylgruppe ist und $R^2$ eine 4-(Pyrid-2-yl)pyrazol-1-yl-Gruppe ist, nämlich (4S,2R,3R,5R)-5-(Hydroxymethyl)-2-[4-(pyridin-2-yl)pyrazolyl]-6-(n-propylamino)purin-9-yl}oxolan-3,4-diol.

**13.** Verbindung nach Anspruch 5, wobei $R^1$ eine gegebenenfalls substituierte Arylgruppe ist und X eine Alkylengruppe ist.

**14.** Verbindung nach Anspruch 13, wobei X-$R^1$ eine 3-Iodbenzylgruppe ist und $R^2$ eine 4-(4-Methoxyphenyl)pyrazol-1-yl-Gruppe ist, nämlich (4S,2R,3R,5R)-5-(Hydroxymethyl)-2-{2-[4-(4-methoxyphenyl)pyrazolyl]-6-(3-iodbenzylamino)purin-9-yl}oxolan-3,4-diol.

**15.** Verbindung nach Anspruch 1, wobei $R^2$ eine gegebenenfalls substituierte Pyrazol-4-yl-Gruppe ist.

**16.** Verbindung nach Anspruch 15, wobei $R^1$ eine gegebenenfalls substituierte Alkyl- oder gegebenenfalls substituierte Arylgruppe ist, $R^3$ eine Hydroxymethylgruppe ist und X eine kovalente Bindung ist.

**17.** Verbindung nach Anspruch 16, wobei $R^1$ eine Methylgruppe ist, $R^2$ eine 1-Benzylpyrazol-4-yl-Gruppe ist, $R^3$ eine Hydroxymethylgruppe ist und X eine kovalente Bindung ist, nämlich (4S,2R,3R,5R)-5-(hydroxymethyl)-2-{2-[1-benzylpyrazolyl]-6-(methylamino)purin-9-yl}oxolan-3,4-diol.

**18.** Verbindung nach Anspruch 16, wobei $R^1$ eine n-Propylgruppe ist, $R^2$ eine 1-Benzylpyrazol-4-yl-Gruppe ist, $R^3$ eine Hydroxymethylgruppe ist und X eine kovalente Bindung ist, nämlich (4S,2R,3R,5R)-5-(Hydroxymethyl)-2-{2-[1-benzylpyrazolyl]-6-(n-propylamino)purin-9-yl}oxolan-3,4-diol.

**19.** Verbindung nach Anspruch 1, wobei $R^2$ eine $R^4$-Z-Y-C≡C--Gruppe ist.

**20.** Verbindung nach Anspruch 19, wobei $R^4$ eine gegebenenfalls substituierte Phenylgruppe ist und Y eine Alkylengruppe mit 1 bis 3 Kohlenstoffatomen ist.

**21.** Verbindung nach Anspruch 20, wobei $R^4$ eine Phenylgruppe ist, die gegebenenfalls durch eine Methoxy- oder Chlorgruppe substituiert ist, und Y eine Methylengruppe ist.

**22.** Verbindung nach Anspruch 21, wobei $R^1$ eine gegebenenfalls substituierte Alkylgruppe ist, X eine kovalente Bindung ist und $R^3$ eine Hydroxymethylgruppe ist.

**23.** Verbindung nach Anspruch 22, wobei $R^1$ eine Methylgruppe ist, $R^4$ eine Phenylgruppe ist und Z ein Sauerstoffatom ist, nämlich 2-Hydroxymethyl-5-[6-methylamino-2-(3-phenoxypropin-1-yl)purin-9-yl]tetrahydrofuran-3,4-diol.

**24.** Verwendung einer Verbindung nach einem der Ansprüche 1 bis 23 zur Herstellung einer pharmazeutischen Zusammensetzung zum Behandeln eines Erkrankungszustands in einem Säuger, der durch Behandlung mit einem $A_3$-Adenosin-Rezeptor-Agonisten linderbar ist.

**25.** Verwendung nach Anspruch 24, wobei der Erkrankungszustand Krebs ist.

**26.** Verwendung nach Anspruch 24, wobei der Erkrankungszustand Neutropenie ist.

**27.** Pharmazeutische Zusammensetzung, die mindestens ein pharmazeutisch verträgliches Exzipiens und eine therapeutisch wirksame Menge einer Verbindung nach einem der Ansprüche 1 bis 23 umfasst.

**28.** Verfahren zur Herstellung einer Verbindung der Formel I:

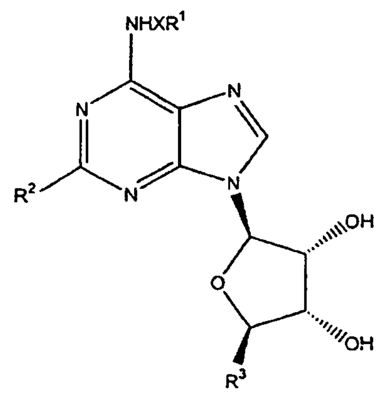

Formel I

worin
$R^1$ eine gegebenenfalls substituierte Niederalkyl-, gegebenenfalls substituierte Cycloalkyl-, gegebenenfalls substituierte Aryl- oder gegebenenfalls substituierte Heteroarylgruppe ist,
X eine kovalente Bindung oder eine gegebenenfalls substituierte Alkylengruppe ist,
$R^2$ eine gegebenenfalls substituierte Pyrazol-1-yl-Gruppe ist und
$R^3$ eine Hydroxymethyl- oder -C(O)-$NR^5R^6$-Gruppe ist,

in der $R^5$ und $R^6$ unabhängig voneinander ein Wasserstoffatom oder eine Niederalkylgruppe sind, umfassend:

ein In-Kontakt-Bringen einer Verbindung der Formel:

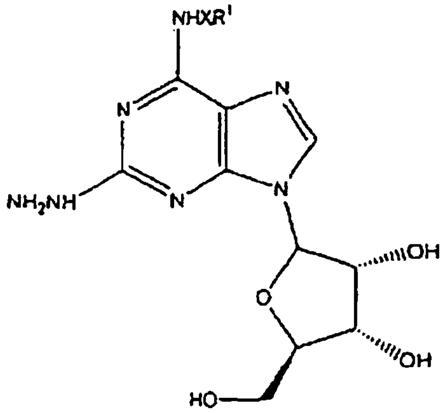

(3)

mit einer Verbindung der Formel:

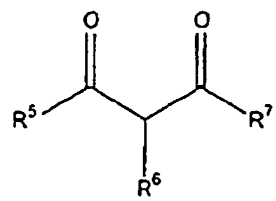

(4)

**29.** Verfahren nach Anspruch 28, wobei die Reaktion in einem inerten Lösungsmittel erfolgt, das aus Methanol, Ethanol, n-Propanol, Isopropanol und t-Butanol ausgewählt ist.

**30.** Verfahren zur Herstellung einer Verbindung der Formel I:

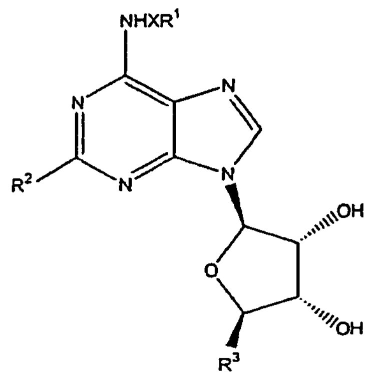

Formel I

in der
$R^1$ eine gegebenenfalls substituierte Niederalkyl-, gegebenenfalls substituierte Cycloalkyl-, gegebenenfalls substituierte Aryl- oder gegebenenfalls substituierte Heteroarylgruppe ist,

X eine kovalente Bindung oder eine gegebenenfalls substituierte Alkylengruppe ist und
$R^2$ eine gegebenenfalls substituierte Pyrazol-4-yl-Gruppe ist,
umfassend:

ein In-Kontakt-Bringen einer Verbindung der Formel:

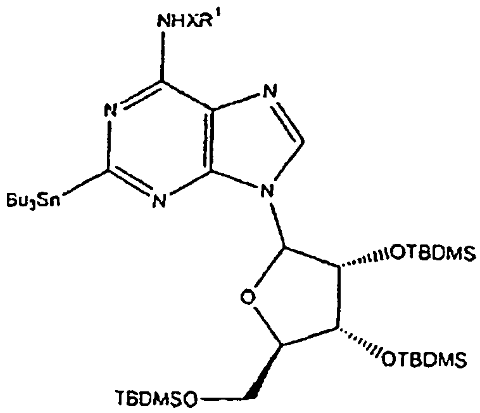

(8)

mit einer Verbindung der Formel:

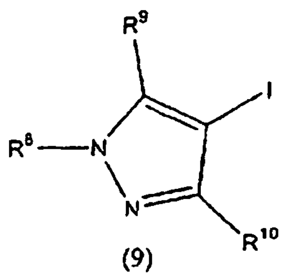

(9)

worin $R^8$, $R^9$ und $R^{10}$ unabhängig voneinander ein Wasserstoffatom oder eine Alkyl-, Alkenyl-, Alkinyl-, Alkoxy-, Cycloalkyl-, Cycloalkenyl-, Acyl-, Acylamino-, Acyloxy-, Amino-, Aminocarbonyl-, Alkoxycarbonylamino-, Azido-, Cyan-, Halogen-, Hydroxy-, Keto-, Thiocarbonyl-, Carboxy-, Carboxyalkyl- (eine Alkylester-), Arylthio-, Heteroaryl-, Heteroarylthio-, Heterocyclylthio-, Thiol-, Alkylthio-, Aryl-, Aryloxy-, Aralkyl-, Heteroaryl-, Aminosulfonyl-, Aminocarbonylamino-, Heteroaryloxy-, Heterocyclyl-, Heterocyclooxy-, Hydroxyamino-, Alkoxyamino-, Nitro-, -SO-Alkyl-, -SO-Aryl-, -SO-Heteroaryl-, $-SO_2$-Alkyl, $-SO_2$-Aryl- und $-SO_2$-Heteroarylgruppe sind,
in Gegenwart eines Palladiumkomplexes und eines Kupfersalzes in einem inerten Lösungsmittel und ein In-Kontakt-Bringen des Produkts mit einer milden Säure.

**31.** Verfahren nach Anspruch 30, wobei der Palladiumkomplex $Pd(PPh_3)_4$ ist, das Kupfersalz CuI ist, das inerte Lösungsmittel N,N-Dimethylformamid ist und die milde Säure Ammoniumfluorid ist.

**32.** Verfahren zur Herstellung einer Verbindung nach Anspruch 1, in der $R^2$ eine $R^4$-Z-Y-C≡C--Gruppe ist, umfassend:

ein In-Kontakt-Bringen in einem inerten Lösungsmittel einer Verbindung der Formel:

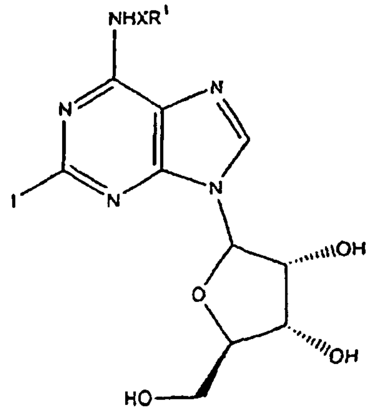

(12)

mit einer Verbindung der Formel:

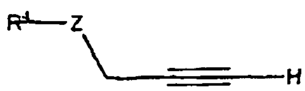

(13)

in Gegenwart einer milden Base, eines Kupfersalzes und eines Palladiumkatalysators.

**33.** Verfahren nach Anspruch 32, wobei das inerte Lösungsmittel N,N-Dimethylformamid ist, die Base Triethylamin ist, das Kupfersalz Kupferiodid ist und der Palladiumkatalysator Dichlorobis(triphenylphosphin)-palladium (II) ist.

**Revendications**

**1.** Composé de formule :

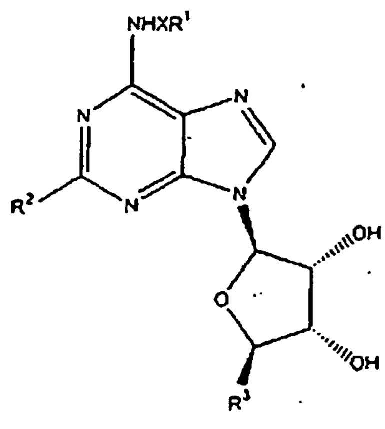

Formule I

dans laquelle :

$R^1$ représente un groupe alkyle inférieur facultativement substitué, cycloalkyle facultativement substitué, aryle facultativement substitué ou hétéroaryle facultativement substitué ;

X représente une liaison covalente ou un groupe alkylène facultativement substitué ;

$R^2$ représente un groupe $R^4$-Z-Y-C≡C- ou un groupe pyrazolyle facultativement substitué ;

dans lequel Y représente un groupe alkylène facultativement substitué, Z représente un atome d'oxygène, un atome de soufre ou un groupe -NH- et $R^4$ représente un groupe aryle facultativement substitué ou un groupe hétéroaryle facultativement substitué ; et

$R^3$ représente un groupe hydroxyméthyle ou -C(O)-$NR^5R^6$ ;

dans lequel $R^5$ et $R^6$ représente indépendamment un atome d'hydrogène ou un groupe alkyle inférieur.

**2.** Composé suivant la revendication 1, dans lequel $R^2$ représente un groupe pyrazole-1-yle facultativement substitué.

**3.** Composé suivant la revendication 2, dans lequel $R^1$ représente un groupe alkyle facultativement substitué ou aryle facultativement substitué et $R^3$ représente un groupe hydroxyméthyle.

**4.** Composé suivant la revendication 3, dans lequel $R^2$ représente un groupe pyrazole-1-yle, substitué avec un substituant alkyle inférieur facultativement substitué, ester, amino-carbonyle, aryle facultativement substitué ou hétéroaryle facultativement substitué.

**5.** Composé suivant la revendication 4, dans lequel le groupe pyrazole-1-yle est substitué avec un substituant phényle facultativement substitué ou benzyle facultativement substitué.

**6.** Composé suivant la revendication 5, dans lequel $R^1$ représente un groupe alkyle inférieur facultativement substitué et X représente une liaison covalente.

**7.** Composé suivant la revendication 6, dans lequel $R^1$ représente un groupe méthyle et $R^2$ représente un groupe 4-(4-méthoxyphényl)-pyrazole-1-yle, à savoir le (4S, 2R, 3R, 5R)-5-(hydroxyméthyl)-2-{2-[4-(4-méthoxyphényl) pyrazolyl)-6-(méthylamino)-purine-9-yl}-oxolane-3,4-diol.

**8.** Composé suivant la revendication 6, dans lequel $R^1$ représente un groupe n-propyle et $R^2$ représente un groupe 4-(4-méthoxyphényl)-pyrazole-1-yle, à savoir le (4S, 2R, 3R, 5R)-5-(hydroxyméthyl)-2-{2-[4-(4-méthoxyphényl)-pyrazolyl]-6-(n-propylamino)-purine-9-yl}-oxolane-3,4-diol.

**9.** Composé suivant la revendication 1, dans lequel $R^1$ représente un groupe méthyle et $R^2$ représente un groupe 4-(4-chlorobenzylaminocarbonyl)pyrazole-1-yle, à savoir le (1-{9-[(4S, 2R, 3R, 5R)-3,4-dihydroxy-5-(hydroxyméthyl)-oxolane-2-yl]-6-(méthylamino)purine-2-yl}-pyrazole-4-yl)-N-(4-chlorobenzyl)-carboxamide.

**10.** Composé suivant la revendication 1, dans lequel $R^1$ représente un groupe méthyle et $R^2$ représente un groupe 4-(4-chlorophénylaminocarbonyl)-pyrazole-1-yle, à savoir le (1-(9-[(4S, 2R, 3R, SR)-3,4-dihydroxy-5-(hydroxyméthyl)-oxolane-2-yl]-6-(méthylamino)-purine-2-yl)-pyrazole-4-yl)-N-(4-chlorophényl)-carboxamide.

**11.** Composé suivant la revendication 4, dans lequel $R^2$ représente un groupe pyrazole-1-yle substitué avec un substituant hétéroaryle facultativement substitué.

**12.** Composé suivant la revendication 11, dans lequel $R^1$ représente un groupe n-propyle et $R^2$ représente un groupe 4-(pyrid-2-yl)-pyrazole-1-yle, à savoir le (4S, 2R, 3R, 5R)-5-(hydroxyméthyl)-2-[4-(pyridine-2-yl)-pyrazolyl]-6-(n-propylamino)-purine-9-yl}-oxolane-3,4-diol.

**13.** Composé suivant la revendication 5, dans lequel $R^1$ représente un groupe aryle facultativement substitué et X représente un groupe alkylène.

**14.** Composé suivant la revendication 13, dans lequel X-$R^1$ représente un groupe 3-iodobenzyle et $R^2$ représente un groupe 4-(4-méthoxyphényl)-pyrazole-1-yle, à savoir le (4S, 2R, 3R, 5R)-5-(hydroxyméthyl)-2-{2-[4-(4-méthoxyphényl)-pyrazolyl]-6-(3-iodobenzylamino)-purine-9-yl}-oxolane-3,4-diol.

**15.** Composé suivant la revendication 1, dans lequel $R^2$ représente un groupe pyrazole-4-yle facultativement substitué.

**16.** Composé suivant la revendication 15, dans lequel $R^1$ représente un groupe alkyle facultativement substitué ou aryle facultativement substitué, $R^3$ représente un groupe hydroxyméthyle et X représente une liaison covalente.

**17.** Composé suivant la revendication 16, dans lequel $R^1$ représente un groupe méthyle, $R^2$ représente un groupe 1-benzylpyrazole-4-yle, $R^3$ représente un groupe hydroxyméthyle et X représente une liaison covalente, à savoir le (4S, 2R, 3R, 5R)-5-(hydroxyméthyl)-2-{2-[1-benzylpyrazolyl]-6-(méthylamino)-purine-9-yl}-oxolane-3,4-diol.

**18.** Composé suivant la revendication 16, dans lequel $R^1$ représente un groupe n-propyle, $R^2$ représente un groupe 1-benzylpyrazole-4-yle, $R^3$ représente un groupe hydroxyméthyle et X représente une liaison covalente, à savoir le (4S, 2R, 3R, 5R)-5-(hydroxyméthyl)-2-{2-[1-benzylpyrazolyl]-6-(n-propylamino)purine-9-yl}-oxolane-3,4-diol.

**19.** Composé suivant la revendication 1, dans lequel $R^2$ représente un groupe $R^4$-Z-Y-C≡C-.

**20.** Composé suivant la revendication 19, dans lequel $R^4$ représente un groupe phényle facultativement substitué et Y représente un groupe alkylène ayant 1 à 3 atomes de carbone.

**21.** Composé suivant la revendication 20, dans lequel $R^4$ représente un groupe phényle facultativement substitué avec un substituant méthoxy ou chloro, et Y représente un groupe méthylène.

**22.** Composé suivant la revendication 21, dans lequel $R^1$ représente un groupe alkyle facultativement substitué, X représente une liaison covalente et $R^3$ représente un groupe hydroxyméthyle.

**23.** Composé suivant la revendication 22, dans lequel $R^1$ représente un groupe méthyle, $R^4$ représente un groupe phényle et Z représente un atome d'oxygène, à savoir le 2-hydroxyméthyl-5-[6-méthylamino-2-(3-phénoxypropyn-1-yl)-purine-9-yl]-tétrahydrofuranne-3,4-diol.

**24.** Utilisation d'un composé suivant l'une quelconque des revendications 1 à 23 pour la préparation d'une composition pharmaceutique destinée au traitement chez un mammifère d'un état pathologique qui peut être soulagé par traitement avec un agoniste du récepteur d'adénosine $A_3$.

**25.** Utilisation suivant la revendication 24, dans laquelle l'état pathologique est le cancer.

**26.** Utilisation suivant la revendication 24, dans laquelle l'état pathologique est la neutropénie.

**27.** Composition pharmaceutique comprenant au moins un excipient pharmaceutiquement acceptable et une quantité thérapeutiquement efficace d'un composé de l'une quelconque des revendications 1 à 23.

**28.** Procédé pour la préparation d'un composé de formule I :

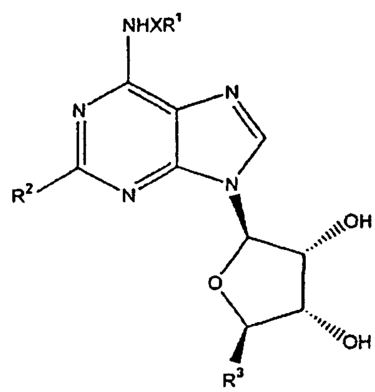

Formule I

dans laquelle :

$R^1$ représente un groupe alkyle inférieur facultativement substitué, cycloalkyle facultativement substitué, aryle

facultativement substitué ou hétéroaryle facultativement substitué ;

X représente une liaison covalente ou un groupe alkylène facultativement substitué ;

$R^2$ représente un groupe pyrazole-1-yle facultativement substitué ; et

$R^3$ représente un groupe hydroxyméthyle ou -C(O)-$NR^5R^6$ ;

dans lequel $R^5$ et $R^6$ représente indépendamment un atome d'hydrogène ou un groupe alkyle inférieur, comprenant :

la mise en contact d'un composé de formule :

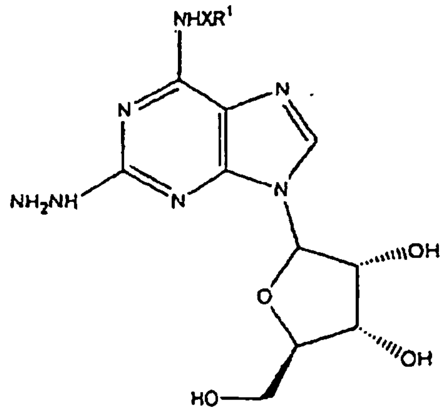

(3)

avec un composé de formule :

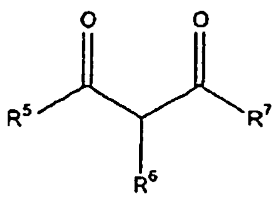

(4)

**29.** Procédé suivant la revendication 28, dans lequel la réaction est conduite dans un solvant inerte choisi entre le méthanol, l'éthanol, le n-propanol, l'isopropanol et le tertiobutanol.

**30.** Procédé pour la préparation d'un composé de formule I :

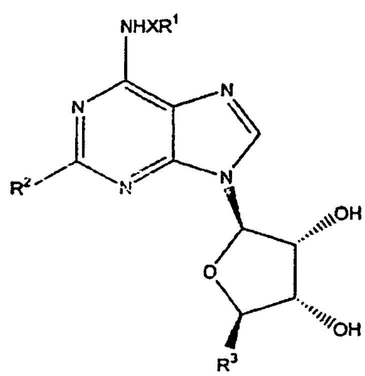

Formule I

dans laquelle :

$R^1$ représente un groupe alkyle inférieur facultativement substitué, cycloalkyle facultativement substitué, aryle facultativement substitué ou hétéroaryle facultativement substitué ;
X représente une liaison covalente ou un groupe alkylène facultativement substitué ; et
$R^2$ représente un groupe pyrazole-4-yle facultativement substitué ;

comprenant :

la mise en contact d'un composé de formule :

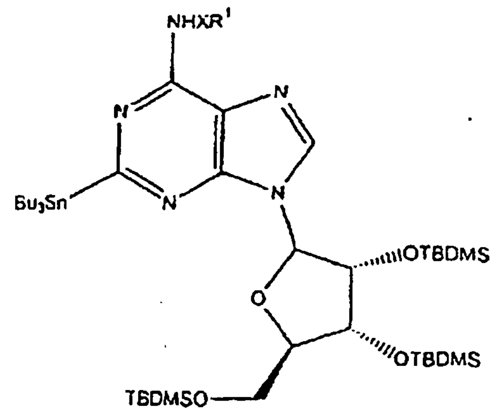

(8)

avec un composé de formule :

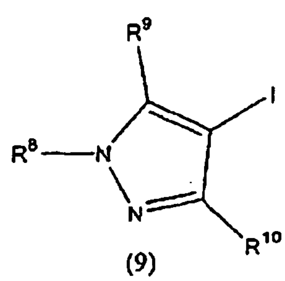

(9)

dans laquelle $R^8$, $R^9$ et $R^{10}$ représentent indépendamment un atome d'hydrogène ou un groupe alkyle, alcényle, alcynyle, alkoxy, cycloalkyle, cycloalcényle, acyle, acylamino, acyloxy, amino, aminocarbonyle, alkoxycarbonylamino, azido, cyano, halogéno, hydroxy, céto, thio-carbonyle, carboxy, carboxyalkyle (un ester alkylique), arylthio, hétéroaryle, héteroarylthio, hétérocyclylthio, thiol, alkylthio, aryle, aryloxy, aralkyle, hétéroaryle, aminosulfonyle, aminocarbonylamino, hétéroaryloxy, hétéro-cyclyle, hétérocyclooxy, hydroxyamino, alkoxyamino, nitro, -SO-alkyle, -SO-aryle, -SO-hétéroaryle, $-SO_2$-alkyle, $-SO_2$-aryle et $-SO_2$-hétéroaryle ;

en présence d'un complexe de palladium et d'un sel de cuivre dans un solvant inerte, et la mise en contact du produit avec un acide faible.

**31.** Procédé suivant la revendication 30, dans lequel le complexe de palladium est $Pd(PPh_3)_4$, le sel de cuivre est CuI, le solvant inerte est le N,N-diméthylformamide et l'acide faible est le fluorure d'ammonium.

**32.** Procédé pour la préparation d'un composé de la revendication 1, dans lequel $R^2$ représente un groupe $R^4$-Z-Y-C≡C- ; comprenant :

la mise en contact dans un solvant inerte d'un composé de formule :

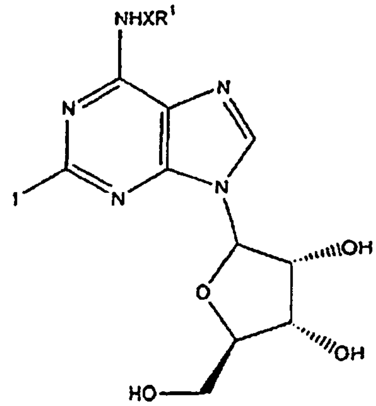

(12)

avec un composé de formule :

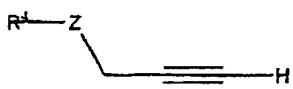

(13)

en présence d'une base faible, d'un sel de cuivre et d'un catalyseur au palladium.

**33.** Procédé suivant la revendication 32, dans lequel le solvant inerte est le N,N-diméthylformamide, la base est la triéthylamine, le sel de cuivre est l'iodure de cuivre et le catalyseur au palladium est le dichlorobis-(triphénylphosphine)palladium (II).